# EUROPEAN PATENT APPLICATION

(11) **EP 4 741 420 A1**
(43) Date of publication of application: **13.05.2026**
(21) Application number: 24832519.3
(22) Date of filing: 28.06.2024
(51) Int. Cl.: C07K 14/605, C07K 19/00, A61K 38/00, A61P 3/04, A23L 33/18, A61K 8/64

(54) **PHARMACEUTICAL COMPOSITION FOR PREVENTING OR TREATING OBESITY COMPRISING GLP ANALOGUE AS ACTIVE INGREDIENT**

(30) Priority: 30.06.2023 KR 20230085302; 27.06.2024 KR 20240084869
(71) Applicant: Huonslab Co., Ltd., Seongnam-si, Gyeonggi-do 13201 (KR)
(72) Inventor: LIM, Chae Young, Seoul 06155 (KR); KIM, Dong Hwan, Yongin-si, Gyeonggi-do 16931 (KR); KWON, Oh Chan, Seoul 08608 (KR)
(74) Representative: Dehns
(86) International application number: PCT/KR2024/009137
(87) International publication number: WO 2025/005743

(57) **Abstract**

The present invention relates to a pharmaceutical composition for preventing or treating obesity, the composition comprising a glucagon-like peptide (GLP) analogue as an active ingredient. The GLP analogue of the present invention is an analog of GLP-1 and GLP-2 and has an extended duration of in vivo activity due to having an increased half-life, and thus was found to have an excellent effect in preventing or treating metabolic diseases. Specifically, the peptide of the present invention reduces the body weight and fat of an individual, reduces the levels of blood fat factors such as blood glucose, total blood cholesterol, high-density lipoproteins, low-density lipoproteins, and triglycerides, and reduces hepatic ASL and AST levels, liver weight, hepatic steatosis levels, and hepatic triglycerides levels, and thus can be effectively used as an excellent agent for preventing or treating metabolic diseases.

## Description

### [Technical Field]

The present invention relates to a pharmaceutical composition for preventing or treating obesity, the composition comprising a GLP analog as an active ingredient.

This application claims priority to and the benefit of Korean Patent Application No. 10-2023-0085302, filed on June 30, 2023, and Korean Patent Application No. 10-2024-0084869, filed on June 27, 2024, the disclosure of which is incorporated herein by reference in its entirety.

### [Background Art]

Obesity, one of the chronic diseases of modern people, has emerged as one of the most serious global health issues, and in 1996, it was classified as a disease by the World Health Organization (WHO). Obesity is steadily increasing due to various reasons, such as dietary habits associated with industrialization and rising income levels, the westernization of eating patterns, and the increased consumption of processed food, and changes in lifestyle.

Obesity is a condition in which metabolic disorders are induced by excessive accumulation of body fat. Although obesity itself may not pose a direct problem, it cannot be overlooked, as it can lead to secondary complications such as hyperlipidemia, hypertension, arteriosclerosis, diabetes, fatty liver, and cardiovascular diseases, as well as social problems resulting from excessive fat accumulation.

For this reason, in 2013, the WHO identified obesity as a new epidemic in the 21^{st} century. Afterwards, as health awareness grew, the world declared war on obesity, and multifaceted research is being conducted to develop obesity drugs, and the obesity treatment market is growing.

The US Food and Drug Administration (FDA) approved sibutramine in 1997 and orlistat in 1999 as obesity drugs that can be used for more than 3 months. In 2006, rimonabant was licensed for use in European countries, including the United Kingdom. However, rimonabant was withdrawn from the market in 2009 due to psychiatric side effects such as suicide, and sibutramine was also withdrawn in 2010 due to concerns about cardiovascular adverse effects. After these incidents, anti-obesity drugs have been facing a period of stagnation.

Meanwhile, glucagon-like peptide (GLP), an appetite control hormone, is a hormone secreted in the small intestine by eating, and it regulates appetite by increasing satiety and blood glucose by inducing insulin secretion in the pancreas. Therefore, various types of GLP analogs have been studied for the treatment of diabetes, etc. However, few have been reported on long-acting GLP analogs that exhibit significant anti-obesity effects.

### [Detailed Description of Invention]

### [Technical Problem]

One object of the present invention is to provide a peptide represented by Formula I below:

HX₂EGX₅FTX₈DX₁₀X₁₁X₁₂X₁₃X₁₄X₁₅X₁₆X₁₇X₁₈X₁₉X₂₀X₂₁FIX₂₄X₂₅LX₂₇ X₂₈X₂₉X₃₀X₃₁X₃₂X₃₃X₃₄X₃₅X₃₆X₃₇X₃₈X₃₉X₄ₒX₄₁X₄₂X₄₃ [Formula I]

In Formula I,
X₂ is Gly or Aib;
X₅ is Thr or Ser;
X₈ is Ser or Asp;
X₁₀ is Leu or Val;
X₁₁ is Ser or Asn;
X₁₂ is Thr or Ser;
X₁₃ is Tyr or Ile;
X₁₄ is Leu or Met;
X₁₅ is Asp or Glu;
X₁₆ is any one selected from the group consisting of Ala, Gly, and Asn;
X₁₇ is Leu or Gln;
X₁₈ and X19 are Ala, or His;
X₂₀ is Arg, or Cys;
X₂₁ is Asp or Glu;
X₂₄ is Asn or Lys;
X₂₅ is Trp or Thr;
X₂₇ is Ile or Val;
X₂₈ is Gln or Lys;
X₂₉ is Thr or Gly;
X₃₀ is Lys or Arg;
X₃₁ is Ile or absent;
X₃₂ is Thr or absent;
X₃₃ is Asp or absent; and
X₃₄ to X₄₃ are absent or Lys.

Another object of the present invention is to provide a pharmaceutical composition for preventing or treating a metabolic disease, the composition comprising the peptide as an active ingredient.

Another object of the present invention is to provide a kit for preventing or treating a metabolic disease, the kit comprising a composition comprising the peptide as an active ingredient and instructions.

Another object of the present invention is to provide a food composition for preventing or improving a metabolic disease, the composition comprising the peptide as an active ingredient.

Another object of the present invention is to provide a cosmetic composition for preventing or improving obesity, the composition comprising the peptide as an active ingredient.

However, technical problems to be solved in the present invention are not limited to the above-described problems, and other problems which are not described herein will be fully understood by those of ordinary skill in the art from the following descriptions.

### [Technical Solution]

The present invention provides a peptide represented by Formula I below:

HX₂EGX₅FTX₈DX₁₀X₁₁X₁₂X₁₃X₁₄X₁₅X₁₆X₁₇X₁₈X₁₉X₂₀X₂₁FIX₂₄X₂₅LX₂₇ X₂₈X₂₉X₃₀X₃₁X₃₂X₃₃X₃₄X₃₅X₃₆X₃₇X₃₈X₃₉X₄₀X₄₁X₄₂X₄₃ [Formula I]

In Formula I,
X₂ is Gly or Aib;
X₅ is Thr or Ser;
X₈ is Ser or Asp;
X₁₀ is Leu or Val;
X₁₁ is Ser or Asn;
X₁₂ is Thr or Ser;
X₁₃ is Tyr or Ile;
X₁₄ is Leu or Met;
X₁₅ is Asp or Glu;
X₁₆ is any one selected from the group consisting of Ala, Gly, and Asn;
X₁₇ is Leu or Gln;
X₁₅ and X19 are Ala, or His;
X₂₀ is Arg, or Cys;
X₂₁ is Asp or Glu;
X₂₄ is Asn or Lys;
X₂₅ is Trp or Thr;
X₂₇ is Ile or Val;
X₂₈ is Gln or Lys;
X₂₉ is Thr or Gly;
X₃₀ is Lys or Arg;
X₃₁ is Ile or absent;
X₃₂ is Thr or absent;
X₃₃ is Asp or absent; and
X₃₄ to X₄₃ are absent or Lys.

In one embodiment of the present invention, X₅ is Thr; X₈, and X₁₁ are Ser; X₁₂ is Thr; X₁₃ is Tyr; X₁₄ is Leu; X₁₅ is Asp; X₁₆ is Ala; X₁₇ is Leu; X₁₉ is Ala; X₂₁ is Asp; X₂₅ is Trp; X₂₇ is Ile; X₂₈ is Gln; X₂₉ is Thr; X₃₀ is Lys; X₃₁ is Ile; X₃₂ is Thr; X₃₃ is Asp, X₃₄ to X₄₃, or X₃₆ to X₄₃ may be absent, but is not limited thereto.

In one embodiment of the present invention, X₁₀ is Leu, X₁₈ is Ala, and X₃₄ and X₃₅ may be Lys, but is not limited thereto.

In one embodiment of the present invention, when X₂₀ is Cys, Cys may be conjugated with polyethylene glycol (PEG), but is not limited thereto.

In one embodiment of the present invention, any one or more amino acids selected from the group consisting of X₂₄ and X₃₅ to X₄₃ may have a fatty acid bound thereto, but is not limited thereto.

In one embodiment of the present invention, the fatty acid may be a C₁₆ to C₂₀ fatty acid, but is not limited thereto.

In one embodiment of the present invention, the fatty acid may be any one selected from the group consisting of palmitic acid, octadecanedioic acid, eicosanedioic acid, margaric acid, heptadecanedioic acid, hexadecanedioic acid, stearic acid, nonadecylic acid, nonadecanedioic acid, and arachidic acid, but is not limited thereto.

**In** one embodiment of the present invention, the peptide may comprise any one selected from the group consisting of the amino acid sequences represented by SEQ ID NOs: 1 to 9, but is not limited thereto.

The present invention provides a pharmaceutical composition for preventing or treating a metabolic disease, the composition comprising the peptide as an active ingredient.

In one embodiment of the present invention, the metabolic disease may be any one or more selected from the group consisting of obesity, diabetes, non-alcoholic fatty liver disease, hepatic steatosis, dyslipidemia, atherosclerosis, insulin resistance syndrome, and complications thereof, but is not limited thereto.

In one embodiment of the present invention, the peptide may have a long-acting form, but is not limited thereto.

In one embodiment of the present invention, the composition may be administered by any one route selected from the group consisting of subcutaneous, intravenous, and oral administrations, but is not limited thereto.

In one embodiment of the present invention, the composition may activate GLP-1 and GLP-2, but is not limited thereto.

In one embodiment of the present invention, the composition may be characterized by any one or more selected from the group consisting of the following, but is not limited thereto:
a) reducing the body weight and fat of an individual;
b) lowering blood glucose, total blood cholesterol, high-density lipoprotein, low-density lipoprotein, and triglycerides; and
c) decreasing hepatic ASL and AST levels, liver weight, a hepatic steatosis grade, and a hepatic triglyceride level..

The present invention provides a kit for preventing or treating a metabolic disease, the kit comprising a composition comprising the peptide as an active ingredient and instructions.

The present invention provides a food composition for preventing or improving a metabolic disease, the composition comprising the peptide as an active ingredient.

In one embodiment of the present invention, the food may be a health functional food, but is not limited thereto.

The present invention provides a cosmetic composition for preventing or improving obesity, the composition comprising the peptide as an active ingredient.

Additionally, the present invention provides a method for preventing, alleviating, or treating a metabolic disease, the method comprising administering a pharmaceutically effective amount of the peptide or a composition comprising the peptide as an active ingredient to a subject in need thereof.

Additionally, the present invention provides a use of the peptide or a composition comprising the peptide as an active ingredient for preventing, alleviating, or treating a metabolic disease.

Additionally, the present invention provides a use of the peptide or a composition comprising the peptide as an active ingredient for preparing a preparation for preventing, alleviating, or treating a metabolic disease.

### [Advantageous Effects]

A pharmaceutical composition for preventing or treating obesity comprising a GLP (glucagon like peptide) analog as an active ingredient is an analog of GLP-1 and GLP-2, and it has been confirmed that the composition exhibits excellent effects in preventing or treating a metabolic disease due to an increased half-life, which results in a prolonged in vivo activity duration. Specifically, the peptide of the present invention may be usefully employed as an excellent agent for preventing or treating a metabolic disease in that it not only reduced the body weight and fat of an individual and decreased levels of blood glucose, total blood cholesterol, high-density lipoprotein, low-density lipoprotein, and triglycerides as blood lipid factors, but also decreased levels of hepatic ASL and AST, liver weight, a hepatic steatosis grade, and a hepatic triglyceride level.

### [Description of Drawings]

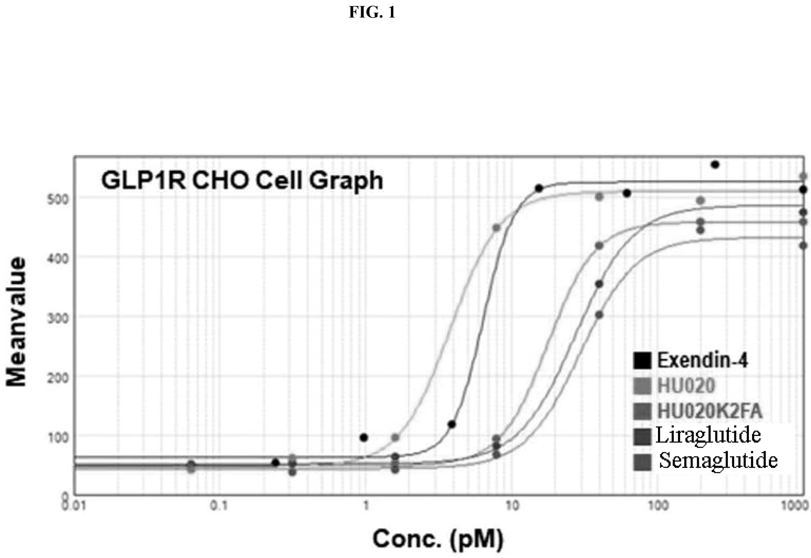

FIG. 1 is a graph showing the excellent GLP-1 activation effect of HU020K2FA (in order based on the peak point, Exendin-4, HU020, liraglutide, HU020K2FA, and semaglutide).

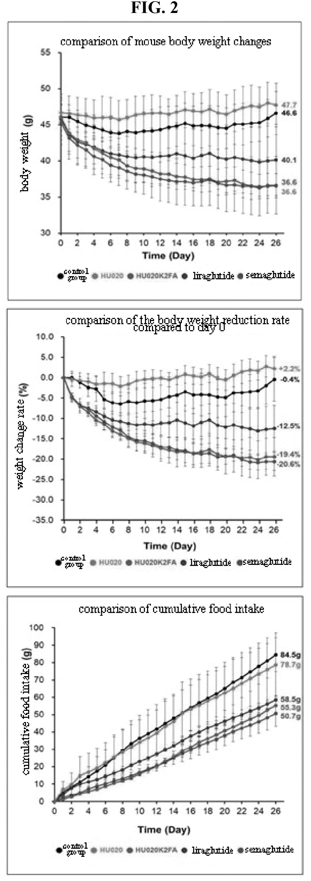

FIG. 2 is a graph showing the effects of reducing body weight and food intake after administering HU020K2FA to an obese mouse model (comparison of mouse body weight changes: in order based on the peak point, HU020, control group, liraglutide, HU020K2FA = semaglutide) (comparison of the body weight reduction rate compared to day 0: in order based on the peak point, HU020, control group, liraglutide, HU020K2FA, semaglutide) (comparison of cumulative food intake: in order based on the peak point, control group, HU020, liraglutide, HU020K2FA, semaglutide).

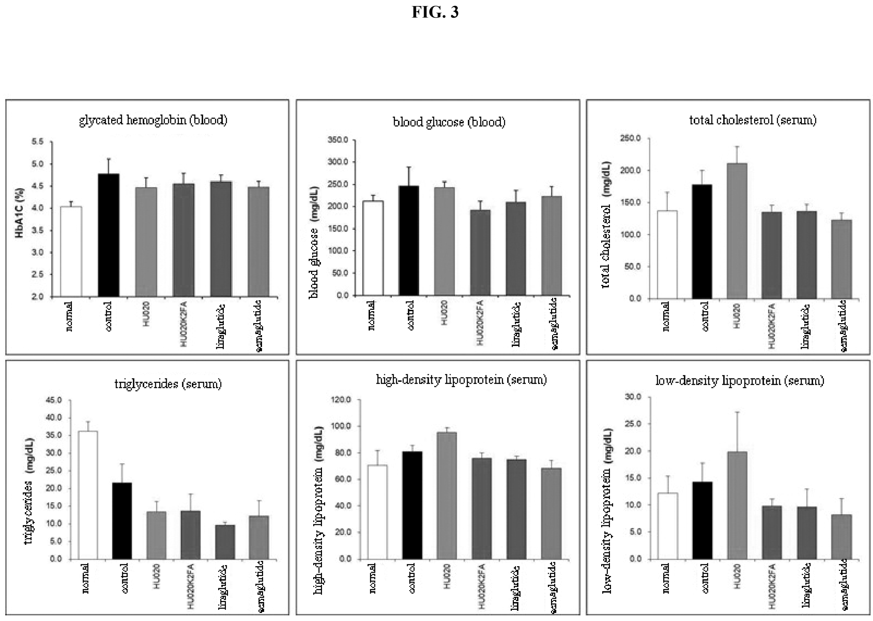

FIG. 3 shows biochemical experiment results obtained after administering HU020K2FA to an obese mouse model.

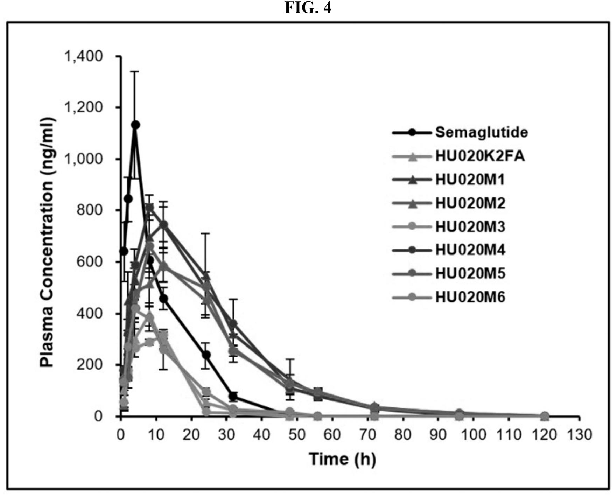

FIG. 4 is a graph showing the mouse PK experiment results analyzed after subcutaneous administration of HU020K2FA and HU020M1 to HU020M6 at 0.2 mg/kg peptide to mice (in order based on the peak point, Semaglutide, HU020M4, HU020M1, HU020M5, HU020M2, HU020M6, HU020K2FA, and HU020M3).

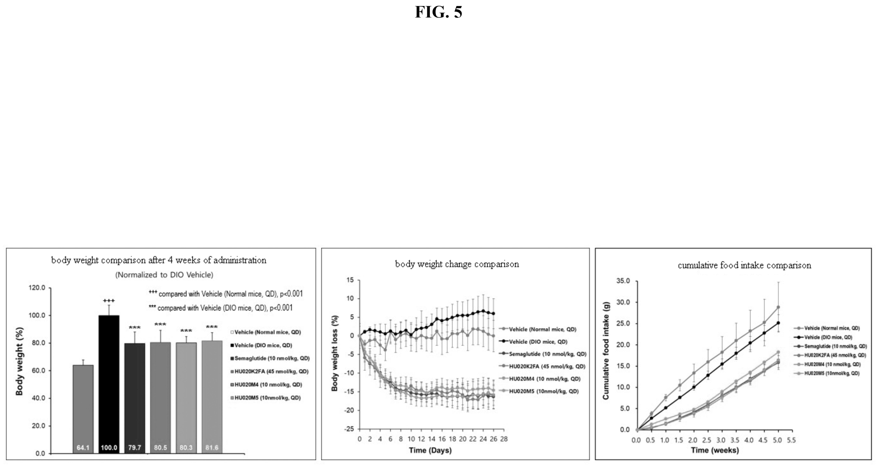

FIG. 5 shows results comparing body weight changes and cumulative food intake after administering HU020K2FA, HU020M4, and HU020M5 to an obese mouse model (comparison of mouse body weight changes: in order based on the peak point, Vehicle (DIO mice, QD), Vehicle (Normal mice, QD), HU020M5 (10 nmol/kg, QD), HU020M4 (10 nmol/kg, QD), HU020K2FA (45 nmol/kg, QD), Semaglutide (10 nmol/kg, QD)) (comparison of cumulative food intake: in order based on the peak point, Vehicle (Normal mice, QD), Vehicle (DIO mice, QD), HU020M5 (10 nmol/kg, QD), HU020M4 (10 nmol/kg, QD), HU020K2FA (45 nmol/kg, QD), Semaglutide (10 nmol/kg, QD)).

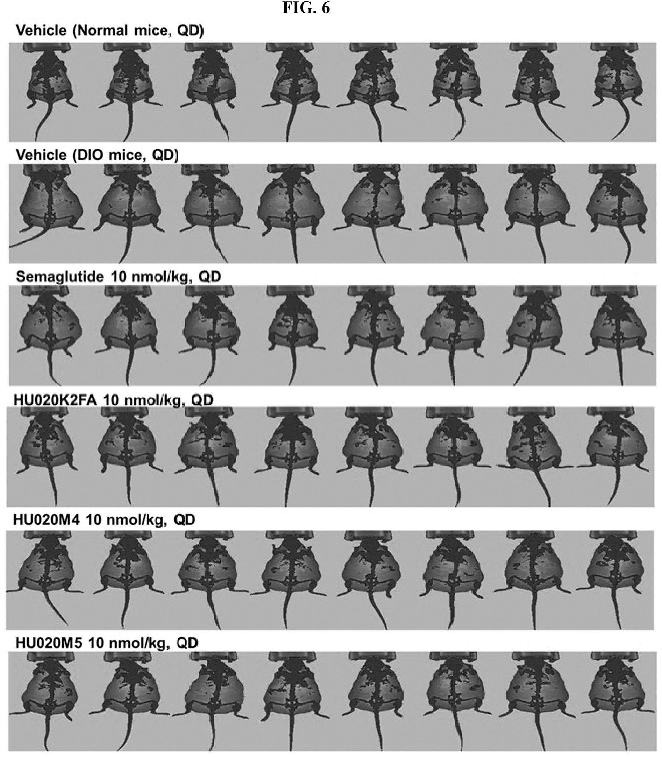

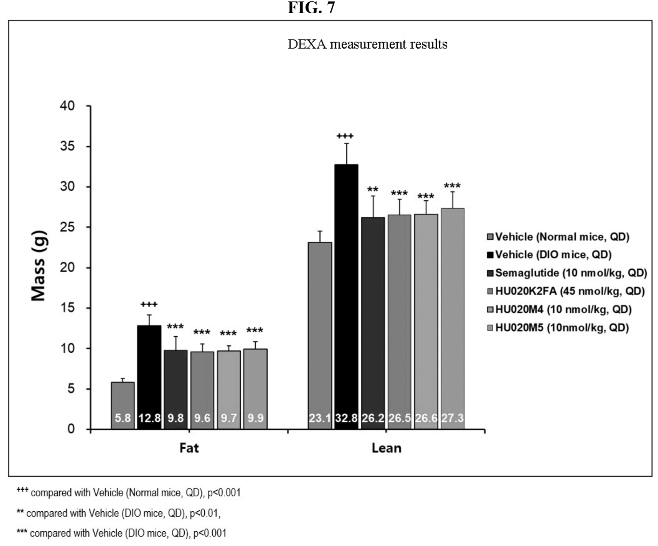

FIGs. 6 and 7 are, respectively, photographs and graphs obtained by DEXA scanning after administering HU020K2FA, HU020M4, and HU020M5 to an obese mouse model (in order from the left, Vehicle (Normal mice, QD), Vehicle (DIO mice, QD), Semaglutide (10 nmol/kg, QD), HU020K2FA (45 nmol/kg, QD), HU020M4 (10 nmol/kg, QD), HU020M5 (10 nmol/kg, QD)).

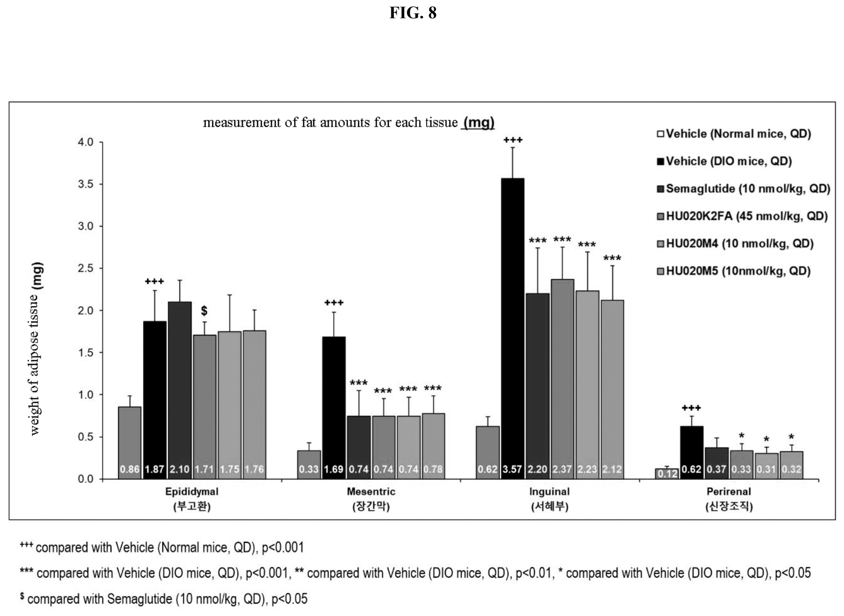

FIG. 8 shows results of measuring fat amounts in each tissue after administering HU020K2FA, HU020M4, and HU020M5 to an obese mouse model (in order from the left, Vehicle (Normal mice, QD), Vehicle (DIO mice, QD), Semaglutide (10 nmol/kg, QD), HU020K2FA (45 nmol/kg, QD), HU020M4 (10 nmol/kg, QD), HU020M5 (10 nmol/kg, QD)).

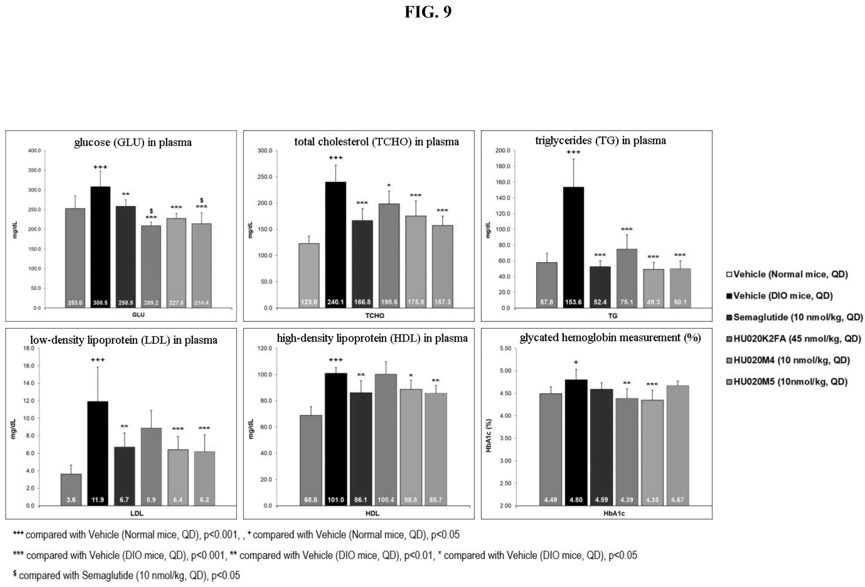

FIG. 9 shows results of analyzing levels of obesity-related factors in plasma after administering HU020K2FA, HU020M4, and HU020M5 to an obese mouse model (in order from the left, Vehicle (Normal mice, QD), Vehicle (DIO mice, QD), Semaglutide (10 nmol/kg, QD), HU020K2FA (45 nmol/kg, QD), HU020M4 (10 nmol/kg, QD), HU020M5 (10 nmol/kg, QD)).

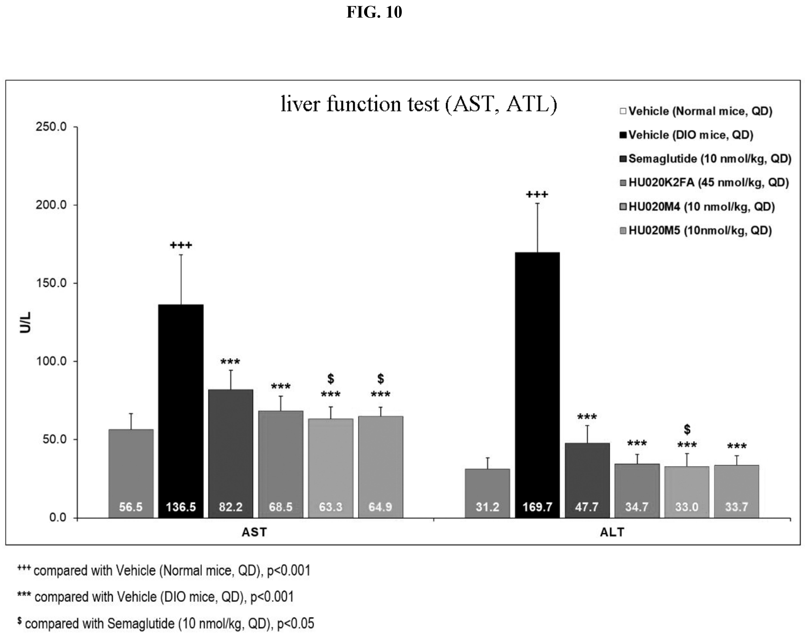

FIG. 10 shows results of analyzing AST/ALT levels, which are hepatic injury markers, after administering HU020K2FA, HU020M4, and HU020M5 to an obese mouse model (in order from the left, Vehicle (Normal mice, QD), Vehicle (DIO mice, QD), Semaglutide (10 nmol/kg, QD), HU020K2FA (45 nmol/kg, QD), HU020M4 (10 nmol/kg, QD), HU020M5 (10 nmol/kg, QD)).

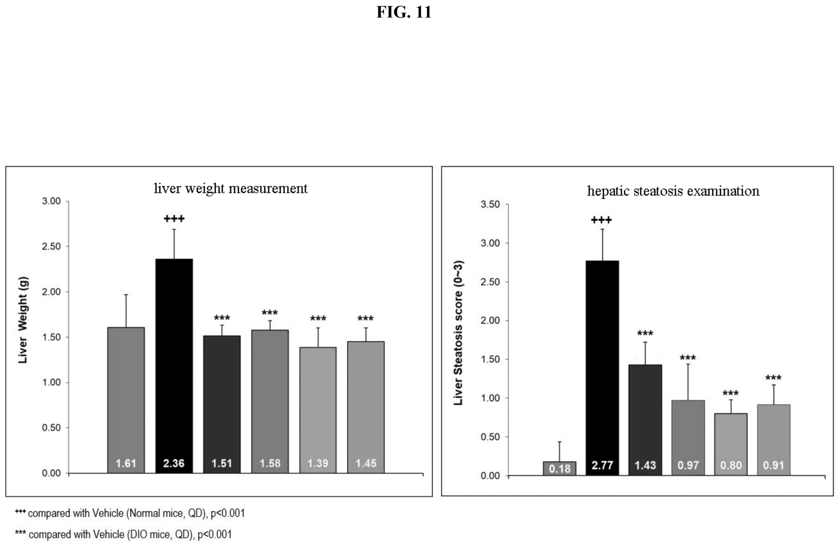

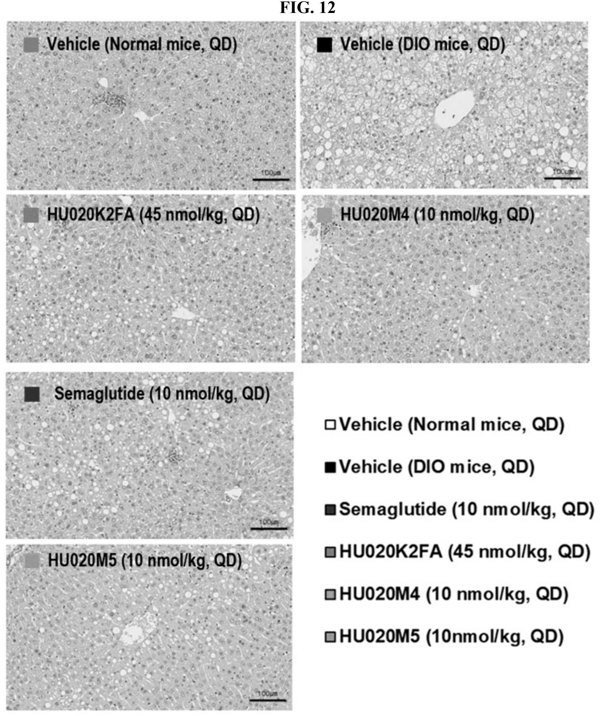

FIGs. 11 and 12 are graphs and photographs, respectively, showing liver weight and hepatic steatosis test results after administering HU020K2FA, HU020M4, and HU020M5 to an obese mouse model (in order from the left, Vehicle (Normal mice, QD), Vehicle (DIO mice, QD), Semaglutide (10 nmol/kg, QD), HU020K2FA (45 nmol/kg, QD), HU020M4 (10 nmol/kg, QD), HU020M5 (10 nmol/kg, QD)).

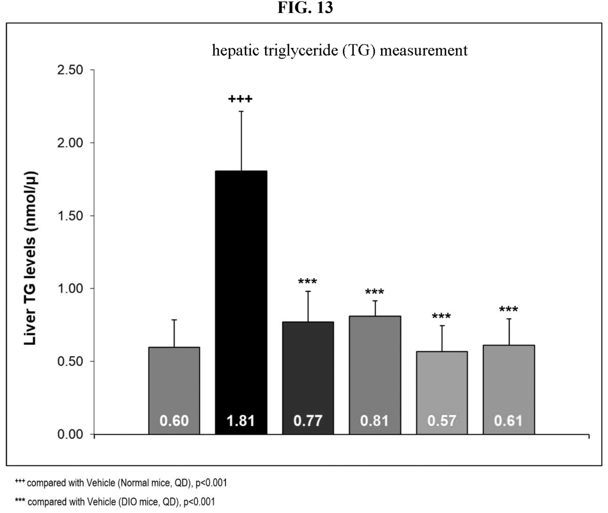

FIG. 13 shows results of measuring hepatic triglycerides after administering HU020K2FA, HU020M4, and HU020M5 to an obese mouse model (in order from the left, Vehicle (Normal mice, QD), Vehicle (DIO mice, QD), Semaglutide (10 nmol/kg, QD), HU020K2FA (45 nmol/kg, QD), HU020M4 (10 nmol/kg, QD), HU020M5 (10 nmol/kg, QD)).

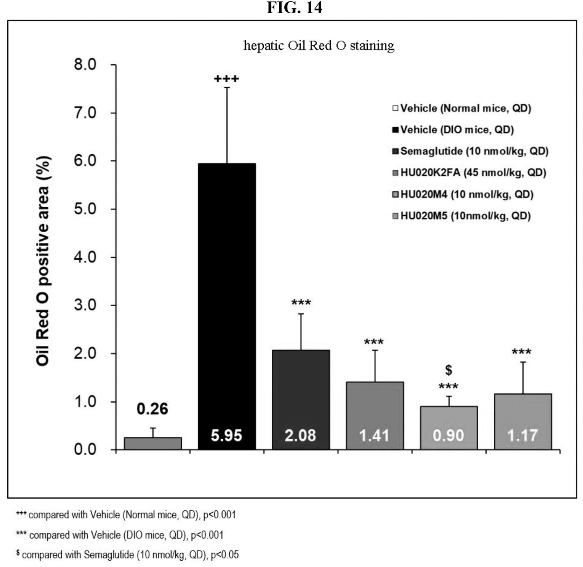

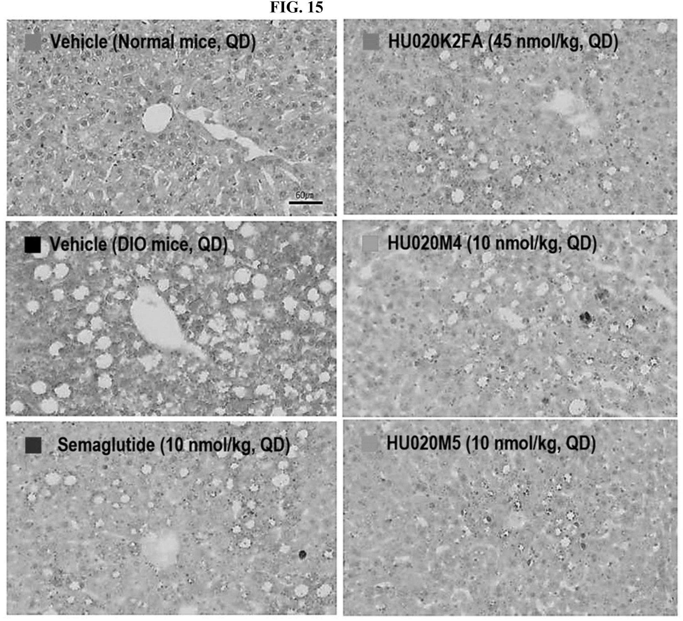

FIGs. 14 and 15 are graphs and photographs, respectively, showing results of Oil Red O staining analysis of the liver after administering HU020K2FA, HU020M4, and HU020M5 to an obese mouse model (in order from the left, Vehicle (Normal mice, QD), Vehicle (DIO mice, QD), Semaglutide (10 nmol/kg, QD), HU020K2FA (45 nmol/kg, QD), HU020M4 (10 nmol/kg, QD), HU020M5 (10 nmol/kg, QD)).

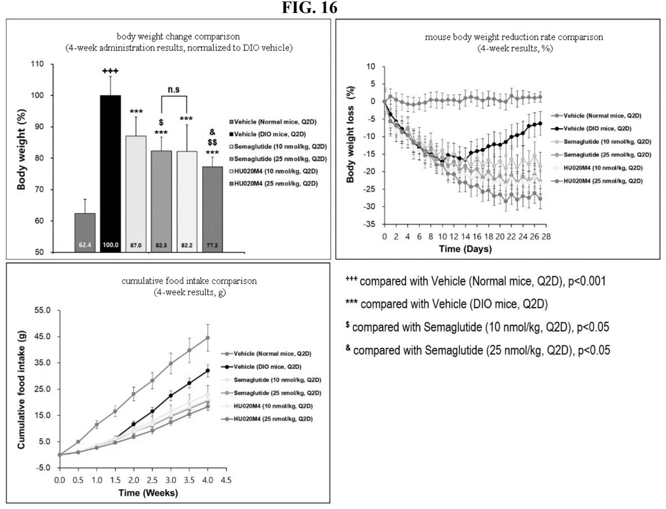

FIG. 16 shows results of comparing body weight changes and cumulative food intake after administering HU020M4 once every two days to an obese mouse model (comparison of body weight changes: in order from the left, Vehicle (Normal mice, Q2D), Vehicle (DIO mice, Q2D), Semaglutide (10 nmol/kg, Q2D), Semaglutide (25 nmol/kg, Q2D), HU020M4 (10 nmol/kg, Q2D), HU020M4 (25 nmol/kg, Q2D)) (comparison of mouse body weight reduction rate: in order based on the peak point, Vehicle (Normal mice, Q2D), Vehicle (DIO mice, Q2D), Semaglutide (10 nmol/kg, Q2D), Semaglutide (25 nmol/kg, Q2D), HU020M4 (10 nmol/kg, Q2D), HU020M4 (25 nmol/kg, Q2D)) (comparison of cumulative food intake: in order based on the peak point, Vehicle (Normal mice, Q2D), Vehicle (DIO mice, Q2D), Semaglutide (10 nmol/kg, Q2D), HU020M4 (10 nmol/kg, Q2D), Semaglutide (25 nmol/kg, Q2D), HU020M4 (25 nmol/kg, Q2D)).

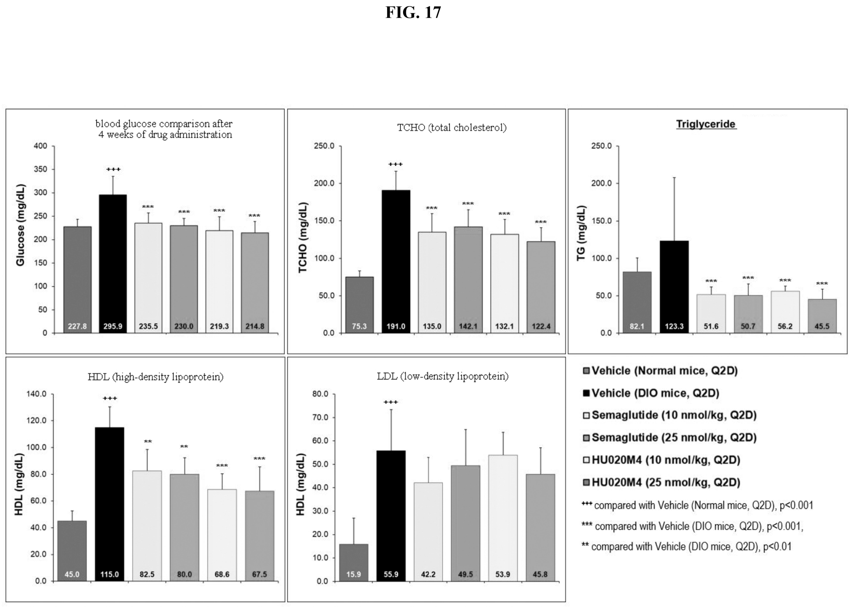

FIG. 17 shows results of analyzing levels of obesity-related factors in plasma after administering HU020M4 once every two days to an obese mouse model (in order from the left, Vehicle (Normal mice, Q2D), Vehicle (DIO mice, Q2D), Semaglutide (10 nmol/kg, Q2D), Semaglutide (25 nmol/kg, Q2D), HU020M4 (10 nmol/kg, Q2D), HU020M4 (25 nmol/kg, Q2D)).

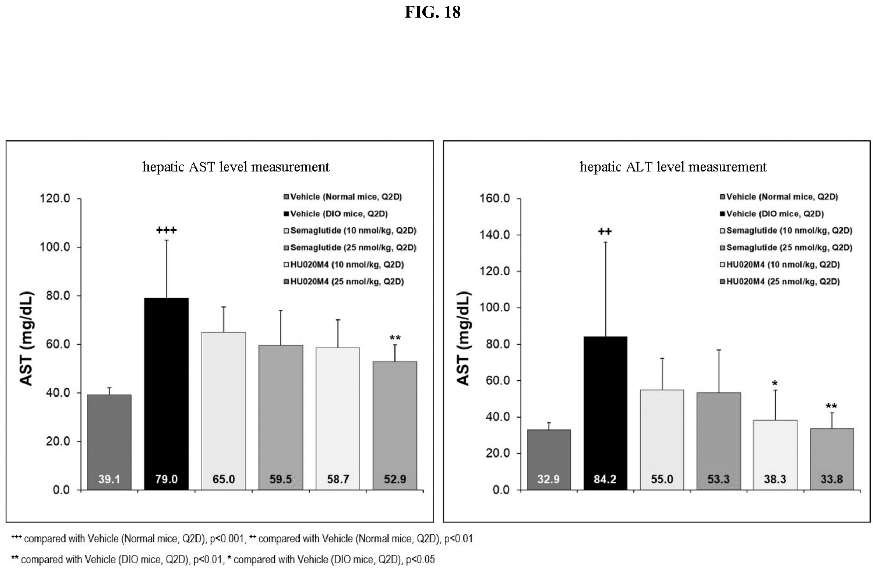

FIG. 18 shows results of analyzing AST/ALT levels, which are hepatic injury markers, after administering HU020M4 once every two days to an obese mouse model (in order from the left, Vehicle (Normal mice, Q2D), Vehicle (DIO mice, Q2D), Semaglutide (10 nmol/kg, Q2D), Semaglutide (25 nmol/kg, Q2D), HU020M4 (10 nmol/kg, Q2D), HU020M4 (25 nmol/kg, Q2D)).

### [Best Mode]

In the present invention, "GLP analog" may refer to a peptide that is a variant comprising a sequence similar to any one or more of GLP, GLP-1, or GLP-2, or a compound. GLP, GLP-1, and GLP-2 all have a characteristic in that they may not be used as themselves. In particular, GLP-1 has insufficient intrinsic activity, and two cleaved naturally occurring peptides, GLP-1 (7-37)OH and GLP-1 (7-36)NH2, are rapidly eliminated in vivo, resulting in a very short in vivo half-life, so that the usefulness of therapies involving GLP-1 peptides has been limited. In particular, endogenously generated dipeptidyl peptidase-3 (DPP-3) inactivates GLP-1 peptides by removing the N-terminal histidine (7th) residue and alanine (8th) residue of GLP-1, and this is known to be a major cause of the short in vivo half-life. That is, the short half-life (t1/2 < 2 minutes) of GLP-1 resulting from degradation by DPP-3 interferes with the application of GLP-1 for treating insulin-resistant type 2 diabetes.

Currently, representative GLP-1 analogs, exenatide, lixisenatide, and liraglutide, are commercially available under the trade names Byetta Pen, Lyxumia Pen, and Victoza Pen, respectively, and are administered subcutaneously to the abdomen, thigh, or upper arm once or twice daily. These commercially available GLP-1 analog subcutaneous injections are not easy to store, and the process of disinfecting and injecting at a fixed time every day is complicated, resulting in poor dosing convenience, and they have disadvantages in that rash and swelling may occur at the injection site.

On the other hand, oral dosage formulations may improve the quality of life of diabetic patients who require continuous administration of therapeutics and may reduce healthcare costs by alleviating the inconvenience of storage and dosing of subcutaneous injections.

Therefore, in order to utilize the excellent activity of GLP and its analogs, there is a high need for improvements in half-life, storage methods, and dosing intervals, and to address this, the inventors of the present invention developed and completed a long-acting GLP analog as a result of repeated research.

Therefore, the present invention provides a peptide represented by Formula I below:

HX₂EGX₅FTX₈DX₁₀X₁₁X₁₂X₁₃X₁₄X₁₅X₁₆X₁₇X₁₈X₁₉X₂₀X₂₁FIX₂₄X_{2 5}LX₂₇X₂₈X₂₉X₃₀X₃₁X₃₂X₃₃X₃₄X₃₅X₃₆X₃₇X₃₈X₃₉X₄ₒX₄₁X₄₂X₄₃ [Formula I]

In Formula I,
X₂ is Gly or Aib;
X₅ is Thr or Ser;
X₈ is Ser or Asp;
X₁₀ is Leu or Val;
X₁₁ is Ser or Asn;
X₁₂ is Thr or Ser;
X₁₃ is Tyr or Ile;
X₁₄ is Leu or Met;
X₁₅ is Asp or Glu;
X₁₆ is any one selected from the group consisting of Ala, Gly, and Asn;
X₁₇ is Leu or Gln;
X₁₈ and X19 are Ala, or His;
X₂₀ is Arg, or Cys;
X₂₁ is Asp or Glu;
X₂₄ is Asn or Lys;
X₂₅ is Trp or Thr;
X₂₇ is Ile or Val;
X₂₈ is Gln or Lys;
X₂₉ is Thr or Gly;
X₃₀ is Lys or Arg;
X₃₁ is Ile or absent;
X₃₂ is Thr or absent;
X₃₃ is Asp or absent; and
X₃₄ to X₄₃ are absent or Lys.

In Formula I of the present invention, the peptide is designed to activate both GLP-1 and GLP-2, and nine sequences essential for GLP-1 activation are fixed.

The GLP analog of the present invention may have X₃₄ to X₄₃ absent or as Lys in Formula I, and in this case, the half-life of the GLP analog of the present invention may vary depending on the number of Lys residues among the nine amino acids of X₃₅ to X₄₃. For example, in one embodiment of the present invention, the GLP analog may have X₃₄ and X₃₅ as Lys and X₃₆ to X₄₃ absent, or X₃₄ to X₄₃ may be absent. In this case, when one or more Lys residues are comprised, compared to when all Lys residues are absent, the half-life may increase, thereby prolonging the in vivo activity duration and providing excellent anti-obesity effects, but is not limited thereto.

Therefore, the present invention may provide a peptide represented by Formula II below according to the number of Lys residues at the C-terminus:

HX₂EGX₅FTX₈DX₁₀X₁₁X₁₂X₁₃X₁₄X₁₅X₁₆X₁₇X₁₈X₁₉X₂₀X₂₁FIX₂₄X_{2 5}LX₂₇X₂₈X₂₉X₃₀X₃₁X₃₂X₃₃X₃₄X₃₅ [Formula II].

In one embodiment of the present invention, X₅ is Thr; X₈, and X₁₁ are Ser; X₁₂ is Thr; X₁₃ is Tyr; X₁₄ is Leu; X₁₅ is Asp; X₁₆ is Ala; X₁₇ is Leu; X₁₉ is Ala; X₂₁ is Asp; X₂₅ is Trp; X₂₇ is Ile; X₂₈ is Gln; X₂₉ is Thr; X₃₀ is Lys; X₃₁ is Ile; X₃₂ is Thr; X₃₃ is Asp, X₃₄ to X₄₃, and X₃₆ to X₄₃ may be absent, but is not limited thereto. When the peptide of the present invention is represented by Formula II, X₃₄ to X₃₅ may be absent, but is not limited thereto. This is a sequence commonly comprised in the peptide of the present invention in addition to amino acids essential for GLP-1 and GLP-2 activation.

Therefore, the present invention may provide a peptide represented by Formula III below:

HX₂EGTFTSDX₁₀STYLDALX₁₈AX₂₀DFIX₂₄WLIQTKITDX₃₄X₃₅ [Formula III].

Therefore, the peptide of the present invention may be represented by the above-described Formula I to Formula III.

In one embodiment of the present invention, X₁₀ is Leu, X₁₈ is Ala, and X₃₄ and X₃₅ may be Lys, but is not limited thereto.

In the peptide sequence of the present invention, the 2nd, 10th, 18th, 20th, 24th, 34th, and 35th amino acids from the N-terminus may be amino acids located at positions that, based on the GLP-1 and GLP-2 amino acid sequences, were identified by the inventors of the present invention as having particularly excellent metabolic disease treatment activity according to their research, and it was confirmed through specific experiments that, depending on the amino acids of the present invention, PEG conjugation, or fatty acid conjugation at the corresponding positions, the treatment effect is superior compared to existing GLP and GLP analogs.

The 35 basic sequences of HU020K2FA, which is the peptide represented by SEQ ID NO: 1 of the present invention, may be composed of (1) seven amino acid sequences present only in GLP-1, (2) fourteen amino acids present only in GLP-2, (3) ten amino acids common to GLP-1 and GLP-2, and (4) four amino acids that are self-mutation amino acids. Specifically, among the sixteen GLP-1 amino acids, nine amino acids are common to GLP-1/2, so the amino acids present only in GLP-1 correspond to seven, and the amino acids identical to those of GLP-2 correspond to a total of twenty-four. That is, HU020K2FA may exhibit significantly high sequence homology with GLP-2, but is not limited thereto. Additionally, HU020K2FA of the present invention may exhibit about 57% sequence identity with the GLP-1 amino acid sequence, and the amino acids may represent a portion of the sequence identical to the GLP-1 amino acid sequence, but is not limited thereto. Therefore, HU020K2FA of the present invention may exhibit about 70% sequence identity with the GLP-2 amino acid sequence, and the following amino acids may represent a portion of the sequence identical to the GLP-2 amino acid sequence, but is not limited thereto.

In the present invention, the terms "protein," "polypeptide," or "peptide" are used interchangeably and refer to polymers of amino acid residues, as generally found in proteins in their natural state.

The long-acting GLP analog of the present invention may be characterized in that it is an analog based on GLP and has an increased half-life, resulting in an excellent in vivo activity duration. Three methods may be applied to produce such a long-acting GLP analog.

The first method may be a method of conjugating a fatty acid to Lys among amino acids including the C-terminus of the GLP analog. When a fatty acid is conjugated, albumin-binding affinity may increase, resulting in an increased half-life, and the effect may vary depending on the carbon number of the fatty acid and the type of fatty acid.

In one embodiment of the present invention, any one or more amino acids selected from the group consisting of X₂₄ and X₃₅ to X₄₃ may have a fatty acid bound thereto, but is not limited thereto. At this time, as described above, the amino acid to which a fatty acid is linked may be Lys.

In one embodiment of the present invention, the fatty acid may be a C₁₆ to C₂₀ fatty acid, but is not limited thereto.

In one embodiment of the present invention, the fatty acid may be any one selected from the group consisting of palmitic acid, octadecanedioic acid, eicosanedioic acid, margaric acid, heptadecanedioic acid, hexadecanedioic acid, stearic acid, nonadecylic acid, nonadecanedioic acid, and arachidic acid, but is not limited thereto.

In the present invention, C16 to C20 fatty acids may be C16, C18, and C20 fatty acids, and preferably may be palmitic acid, octadecanedioic acid, or eicosanedioic acid, respectively, but is not limited thereto. In one embodiment of the present invention, it was confirmed that GLP-1 and GLP-2 activities were increased according to fatty acid conjugation, and it was confirmed that the type or length of the fatty acid affected persistence or anti-obesity effects. Although characteristics depending on the type or length of the fatty acid were identified, it was confirmed with specific data that GLP-1 and GLP-2 activities and anti-obesity-related activities were equal to or greater than those in the absence of a fatty acid, regardless of the type of fatty acid (see, e.g., Example 5).

As a second method for increasing the half-life of the GLP analog of the present invention, PEG conjugation may be used.

In this case, it is important to conjugate PEG to a site that does not affect activity so as to increase the half-life and stability while maintaining a high level of anti-obesity therapeutic effect, and in one embodiment of the present invention, when the X₂₀ is Cys, PEG (polyethylene glycol) may be conjugated to Cys, but is not limited thereto.

At this time, the method of binding PEG and X₂₀ is not limited. For example, they may be bound through a linker or may be directly bound, and the binding refers to all types of binding including covalent bonds and noncovalent bonds, and may be directly linked by covalent bonding due to known genetic recombination techniques, and may be bound through all methods generally performed in the art.

Third, to produce the long-acting GLP analog of the present invention, an amino acid at a specific position may be substituted with Aib (non-coded amino acid, alpha-amino isobutyric acid), and in this case, an effect of increasing the half-life may be obtained by preventing peptide degradation by the DPP-IV enzyme.

The long-acting GLP analog of the present invention may have the second amino acid substituted with Aib, but is not limited thereto.

In one embodiment of the present invention, the peptide may comprise any one selected from the group consisting of the amino acid sequences represented by SEQ ID NOs: 1 to 9, but is not limited thereto. Additionally, the peptide of the present invention may be a sequence composed of any one selected from the group consisting of the amino acid sequences represented by SEQ ID NOs: 1 to 9, but is not limited thereto.

The present invention provides a pharmaceutical composition for preventing or treating a metabolic disease, the composition comprising the peptide as an active ingredient.

In one embodiment of the present invention, the metabolic disease may be any one or more selected from the group consisting of obesity, diabetes, non-alcoholic fatty liver disease, hepatic steatosis, dyslipidemia, atherosclerosis, insulin resistance syndrome, and complications thereof, but is not limited thereto.

In one embodiment of the present invention, the peptide may have a long-acting form, but is not limited thereto.

In the present invention, the term "long-acting" may refer to a form in which in vivo stability is increased, peptide degradation by various enzymes is prevented so that the half-life is increased, the blood retention period is increased, or the duration of activity and bioavailability are increased, but is not limited thereto. In the present invention, it was confirmed that, when the peptide of the present invention was administered once daily as a single dose, it reduced levels of blood obesity-related factors including blood glucose, reduced body weight and fat mass, and also exhibited various lipid-suppression-related effects in the liver. In addition, it was confirmed that the above effects were still excellent even when administered once every two days, and in this case, it was confirmed that excellent effects were exhibited at a lower dose compared to Semaglutide as the control group.

In one embodiment of the present invention, the composition may be administered by any one method selected from the group consisting of subcutaneous, intravenous, and oral administrations, but is not limited thereto.

The GLP analog of the present invention exhibits a long-acting characteristic by applying the above-described three methods, and, compared to existing GLP analog obesity or blood glucose-lowering agents, may have an extended dosing interval to improve dosing convenience, reduce the burden in the body, and further provide an effect of alleviating cost burden.

In one embodiment of the present invention, the composition may activate GLP-1 and GLP-2, but is not limited thereto. In particular, it was confirmed that the peptide of the present invention exhibited especially superior GLP-1 activation effects, and also exhibited remarkable GLP-2 activation effects.

In one embodiment of the present invention, the composition may be characterized by any one or more selected from the group consisting of the following, but is not limited thereto:
a) reducing the body weight and fat of an individual;
b) lowering blood glucose, total blood cholesterol, high-density lipoprotein, low-density lipoprotein, and triglycerides; and
c) decreasing hepatic ASL and AST levels, liver weight, a hepatic steatosis grade, and a hepatic triglyceride level.

The pharmaceutical composition according to the present invention may further comprise a suitable carrier, excipient, and diluent which are commonly used in the preparation of pharmaceutical compositions. The excipient may be, for example, one or more selected from the group consisting of a diluent, a binder, a disintegrant, a lubricant, an adsorbent, a humectant, a film-coating material, and a controlled release additive.

The pharmaceutical composition according to the present invention may be used by being formulated, according to commonly used methods, into a form such as powders, granules, sustained-release-type granules, enteric granules, liquids, eye drops, elixirs, emulsions, suspensions, spirits, troches, aromatic water, lemonades, tablets, sustained-release-type tablets, enteric tablets, sublingual tablets, hard capsules, soft capsules, sustained-release-type capsules, enteric capsules, pills, tinctures, soft extracts, dry extracts, fluid extracts, injections, capsules, perfusates, or a preparation for external use, such as plasters, lotions, pastes, sprays, inhalants, patches, sterile injectable solutions, or aerosols. The preparation for external use may have a formulation such as creams, gels, patches, sprays, ointments, plasters, lotions, liniments, pastes, or cataplasmas.

As the carrier, the excipient, and the diluent that may be comprised in the pharmaceutical composition according to the present invention, lactose, dextrose, sucrose, oligosaccharides, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil may be used.

For formulation, commonly used diluents or excipients such as fillers, thickeners, binders, wetting agents, disintegrants, and surfactants are used.

As additives of tablets, powders, granules, capsules, pills, and troches according to the present invention, excipients such as corn starch, potato starch, wheat starch, lactose, white sugar, glucose, fructose, D-mannitol, precipitated calcium carbonate, synthetic aluminum silicate, dibasic calcium phosphate, calcium sulfate, sodium chloride, sodium hydrogen carbonate, purified lanolin, microcrystalline cellulose, dextrin, sodium alginate, methyl cellulose, sodium carboxymethylcellulose, kaolin, urea, colloidal silica gel, hydroxypropyl starch, hydroxypropyl methylcellulose (HPMC), HPMC 1928, HPMC 2208, HPMC 2906, HPMC 2910, propylene glycol, casein, calcium lactate, and *Primojel*^{®}; and binders such as gelatin, Arabic gum, ethanol, agar powder, cellulose acetate phthalate, carboxymethylcellulose, calcium carboxymethylcellulose, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch, hydroxypropyl cellulose, hydroxypropyl methylcellulose, polyvinyl alcohol, and polyvinylpyrrolidone may be used, and disintegrants such as hydroxypropyl methylcellulose, corn starch, agar powder, methylcellulose, bentonite, hydroxypropyl starch, sodium carboxymethylcellulose, sodium alginate, calcium carboxymethylcellulose, calcium citrate, sodium lauryl sulfate, silicic anhydride, 1-hydroxypropylcellulose, dextran, ion-exchange resin, polyvinyl acetate, formaldehyde-treated casein and gelatin, alginic acid, amylose, guar gum, sodium bicarbonate, polyvinylpyrrolidone, calcium phosphate, gelled starch, Arabic gum, amylopectin, pectin, sodium polyphosphate, ethyl cellulose, white sugar, magnesium aluminum silicate, a di-sorbitol solution, and light anhydrous silicic acid; and lubricants such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, and light anhydrous silicic acid may be used.

As additives of liquids according to the present invention, water, dilute hydrochloric acid, dilute sulfuric acid, sodium citrate, monostearic acid sucrose, polyoxyethylene sorbitol fatty acid esters (twin esters), polyoxyethylene monoalkyl ethers, lanolin ethers, lanolin esters, acetic acid, hydrochloric acid, ammonia water, ammonium carbonate, potassium hydroxide, sodium hydroxide, prolamine, polyvinylpyrrolidone, ethylcellulose, and sodium carboxymethylcellulose may be used.

In syrups according to the present invention, a white sugar solution, other sugars or sweeteners, and the like may be used, and as necessary, a fragrance, a colorant, a preservative, a stabilizer, a suspending agent, an emulsifier, a viscous agent, or the like may be used.

In emulsions according to the present invention, purified water may be used, and as necessary, an emulsifier, a preservative, a stabilizer, a fragrance, or the like may be used.

In suspensions according to the present invention, suspending agents such as acacia, tragacanth, methylcellulose, carboxymethylcellulose, sodium carboxymethylcellulose, microcrystalline cellulose, sodium alginate, hydroxypropyl methylcellulose (HPMC), HPMC 1828, HPMC 2906, HPMC 2910, and the like may be used, and as necessary, a surfactant, a preservative, a stabilizer, a colorant, and a fragrance may be used.

Injections according to the present invention may comprise: solvents such as distilled water for injection, a 0.9% sodium chloride solution, Ringer's solution, a dextrose solution, a dextrose+sodium chloride solution, PEG, lactated Ringer's solution, ethanol, propylene glycol, non-volatile oil-sesame oil, cottonseed oil, peanut oil, soybean oil, corn oil, ethyl oleate, isopropyl myristate, and benzene benzoate; cosolvents such as sodium benzoate, sodium salicylate, sodium acetate, urea, urethane, monoethylacetamide, butazolidine, propylene glycol, the Tween series, amide nicotinate, hexamine, and dimethylacetamide; buffers such as weak acids and salts thereof (acetic acid and sodium acetate), weak bases and salts thereof (ammonia and ammonium acetate), organic compounds, proteins, albumin, peptone, and gums; isotonic agents such as sodium chloride; stabilizers such as sodium bisulfite (NaHSO3) carbon dioxide gas, sodium metabisulfite (Na2S2O5), sodium sulfite (Na2SO3), nitrogen gas (N2), and ethylenediamine tetraacetic acid; sulfating agents such as 0.1% sodium bisulfide, sodium formaldehyde sulfoxylate, thiourea, disodium ethylenediaminetetraacetate, and acetone sodium bisulfite; a pain relief agent such as benzyl alcohol, chlorobutanol, procaine hydrochloride, glucose, and calcium gluconate; and suspending agents such as sodium CMC, sodium alginate, Tween 80, and aluminum monostearate.

In suppositories according to the present invention, bases such as cacao butter, lanolin, Witepsol, polyethylene glycol, glycerogelatin, methylcellulose, carboxymethylcellulose, a mixture of stearic acid and oleic acid, Subanal, cottonseed oil, peanut oil, palm oil, cacao butter + cholesterol, lecithin, lanette wax, glycerol monostearate, Tween or span, imhausen, monolan(propylene glycol monostearate), glycerin, Adeps solidus, buytyrum Tego-G, cebes Pharma 16, hexalide base 95, cotomar, Hydrokote SP, S-70-XXA, S-70-XX75(S-70-XX95), Hydrokote 25, Hydrokote 711, idropostal, massa estrarium (A, AS, B, C, D, E, I, T), masa-MF, masupol, masupol-15, neosuppostal-N, paramount-B, supposiro OSI, OSIX, A, B, C, D, H, L, suppository base IV types AB, B, A, BC, BBG, E, BGF, C, D, 299, suppostal N, Es, Wecoby W, R, S, M, Fs, and tegester triglyceride matter (TG-95, MA, 57) may be used.

Solid preparations for oral administration comprise tablets, pills, powders, granules, capsules, and the like, and such solid preparations are formulated by mixing the composition with at least one excipient, e.g., starch, calcium carbonate, sucrose, lactose, gelatin, and the like. In addition to simple excipients, lubricants such as magnesium stearate and talc are also used.

Examples of liquid preparations for oral administration comprise suspensions, liquids for internal use, emulsions, syrups, and the like, and these liquid preparations may comprise, in addition to simple commonly used diluents, such as water and liquid paraffin, various types of excipients, for example, a wetting agent, a sweetener, a fragrance, a preservative, and the like. Preparations for parenteral administration comprise an aqueous sterile solution, a non-aqueous solvent, a suspension, an emulsion, a freeze-dried preparation, and a suppository. Non-limiting examples of the non-aqueous solvent and the suspension comprise propylene glycol, polyethylene glycol, a vegetable oil such as olive oil, and an injectable ester such as ethyl oleate.

The pharmaceutical composition according to the present invention is administered in a pharmaceutically effective amount. In the present invention, "the pharmaceutically effective amount" refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields.

The composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art.

The pharmaceutical composition of the present invention may be administered to a subject via various routes. All administration methods can be predicted, and the pharmaceutical composition may be administered via, for example, oral administration, subcutaneous injection, intraperitoneal injection, intravenous injection, intramuscular injection, intrathecal (space around the spinal cord) injection, sublingual administration, administration via the buccal mucosa, intrarectal insertion, intravaginal insertion, ocular administration, intra-aural administration, intranasal administration, inhalation, spraying via the mouth or nose, transdermal administration, percutaneous administration, or the like.

The pharmaceutical composition of the present invention is determined depending on the type of a drug, which is an active ingredient, along with various related factors such as a disease to be treated, administration route, the age, gender, and body weight of a patient, and the severity of diseases. Specifically, the effective amount of the composition according to the present invention may vary depending on the age, sex, and body weight of a patient, and generally, 0.001 to 150 mg per 1 kg of body weight, preferably 0.01 to 100 mg, may be administered daily or every other day, or may be administered one to three times per day. However, the dosage may be increased or decreased depending on the administration route, severity of the disease, sex, body weight, age, and the like, and therefore, the above dosage does not limit the scope of the present invention in any way.

As used herein, the "subject" refers to a subject in need of treatment of a disease, and more specifically, refers to a mammal such as a human or a non-human primate, a mouse, a rat, a dog, a cat, a horse, and a cow.

As used herein, the "administration" refers to providing a subject with a predetermined composition of the present invention by using an arbitrary appropriate method. The term "prevention" as used herein means all actions that inhibit or delay the onset of a target disease. The term "treatment" as used herein means all actions that alleviate or beneficially change a target disease and abnormal metabolic symptoms caused thereby via administration of the pharmaceutical composition according to the present invention. The term "alleviation" as used herein means all actions that reduce the degree of parameters related to a target disease, e.g., symptoms via administration of the composition according to the present invention.

The present invention provides a kit for preventing or treating a metabolic disease, the kit comprising a composition comprising the peptide as an active ingredient and instructions. In the present invention, the instructions may describe an increased dosing interval or administration interval according to the increased half-life, but is not limited thereto.

The "kit" of the present invention may further comprise, in addition to the above components, other components, devices, or materials that are ordinarily required for storage, management, enhancement of effects, or administration of the composition of the present invention. Additionally, all components comprised in the kit may be used one or more times without limitation on the number of uses, the order in which each material is used is not limited, and the application of each material may be performed simultaneously or may be performed sequentially.

The kit of the present invention may comprise a container in addition to the above preparation and instructions. The container may serve to package the above components, and may also serve to store and secure them. The material of the container may take the form of, for example, a bottle, tub, sachet, envelope, tube, or ampoule, and may be formed partially or entirely from plastic, glass, paper, foil, wax, or the like. The container may be equipped with a cap that is initially a part of the container or is completely or partially detachable and attachable to the container by mechanical, adhesive, or other means, and may also be equipped with a stopper that allows access to the contents with a syringe needle. The kit may comprise an external package, and the external package may comprise instructions for use of the components, but is not limited thereto.

The present invention provides a food composition for preventing or improving a metabolic disease, the composition comprising the peptide as an active ingredient.

In one embodiment of the present invention, the food may be a health functional food, but is not limited thereto.

In the present invention, the term "food" refers to a natural product or processed product containing one or more nutrients, and preferably refers to a state in which the product can be directly consumed after undergoing a certain processing procedure, and in a conventional sense, comprises all of health functional foods, beverages, food additives, and beverage additives.

In the present invention, the term "functional food" is the same term as food for special health use (FoSHU), and refers to a food with high medical or therapeutic effects that is processed so that, in addition to supplying nutrients, bioregulatory functions are efficiently exhibited, and may be manufactured in the form of tablets, capsules, pills, granules, powders, liquids, flakes, pastes, syrups, gels, jellies, bars, or films. Here, the term "functionality" means obtaining beneficial effects for health use, such as regulating nutrients or exerting physiological actions on the structure and function of the human body. In the present invention, the food may comprise food for inner beauty and health functional food for inner beauty.

In the present invention, there is no particular limitation on the type of the health functional food. Examples of foods to which the composition of the present invention may be added comprise dairy products including ice cream, various soups, beverages, teas, drinks, alcoholic beverages, and vitamin complexes, and in particular, comprise all foods that have been given added value so that the functions of the corresponding foods in the conventional sense act or manifest for a specific purpose, or foods designed to sufficiently exhibit in the body in vivo regulatory functions related to bioprotective rhythm control, disease prevention, and recovery possessed by the food composition.

In the present invention, the term "food additive" refers to a substance used in preparing, processing, or preserving food by being added to, mixed with, or infiltrated into the food or by other methods, and must be harmless to the human body when ingested for a long period as in the case of health functional foods.

When the composition of the present invention is used as a food additive, the food additive may be used by adding the composition of the present invention as it is or together with other foods or food ingredients, and may be appropriately used according to conventional methods.

The mixing amount of the active ingredient may be appropriately determined depending on the intended use (prevention, health, or therapeutic treatment). Generally, in manufacturing foods or beverages, the composition of the present invention may be added in an amount of 15 wt% or less or 10 wt% or less based on the raw material. However, in the case of long-term intake for the purpose of health and hygiene or for health regulation, the amount may be below the above range, and since there is no problem in terms of safety, the active ingredient may also be used in an amount greater than the above range.

In the present invention, the composition may comprise various food auxiliary additives that are acceptable in food science, and may further comprise appropriate carriers, excipients, and diluents that are conventionally used in the manufacture of foods.

Additionally, in addition to the above, the composition of the present invention may contain various nutrients, vitamins, electrolytes, flavoring agents, coloring agents, pectic acid and salts thereof, alginic acid and salts thereof, organic acids, protective colloid thickeners, pH adjusters, stabilizers, preservatives, glycerin, alcohol, and carbonating agents used in carbonated beverages. The composition of the present invention may further contain pulp for producing natural fruit juice, fruit juice beverages, and vegetable beverages. Such components may be used independently or in combination. The proportion of such additives is not highly critical, but is generally selected in the range of 0.01 to 0.20 parts by weight per 100 parts by weight of the composition of the present invention, but is not limited thereto, and may be an optimal or arbitrary amount depending on the type and function of the product in which it is used.

In the present invention, there is no particular limitation on the type of the food. Examples of foods to which the substance may be added comprise meat, sausages, bread, chocolate, candies, snacks, confectionery, pizza, ramen, other noodles, gums, dairy products including ice cream, various soups, beverages, teas, drinks, alcoholic beverages, and vitamin complexes, and may comprise all health functional foods in the conventional sense, but is not limited thereto.

Additionally, the composition according to the present invention may be added to health beverages and may contain, as additional components, various flavoring agents or natural carbohydrates as in conventional beverages. The above natural carbohydrates are monosaccharides such as glucose and fructose, disaccharides such as maltose and sucrose, polysaccharides such as dextrin and cyclodextrin, and sugar alcohols such as xylitol, sorbitol, and erythritol. As sweeteners, natural sweeteners such as thaumatin and stevia extract or synthetic sweeteners such as saccharin and aspartame may be used. The proportion of the natural carbohydrate may generally be about 0.01 to 0.20 g or about 0.04 to 0.10 g per 100 mL of the composition of the present invention, but is not limited thereto, and may be a general amount added in the art, may comprise a maximum range to enhance the efficacy of the composition of the present invention, and may comprise an optimal or arbitrary amount considering the synergistic effects with other substances added together.

The present invention provides a cosmetic composition for preventing or improving obesity, the composition comprising the peptide as an active ingredient.

In the present invention, the term "cosmetic" may be a concept including all articles that beautify the appearance of the human body, but is not limited thereto, and may comprise the broadest meaning used in the art.

The cosmetic composition of the present invention may be used in cosmetics or may be used as a cosmetic additive, and when the composition of the present invention is used as a cosmetic additive, it may be used in producing a cosmetic composition for maintaining cleanliness of hands or feet. Examples comprise soaps (solid soaps, liquid soaps, foam soaps, body soaps, hand soaps, etc.), cleansing foams, and shampoos (hair shampoos, dry shampoos, etc.), but is not limited thereto.

The cosmetic composition of the present invention may be formulated in any form conventionally prepared in the art, and for example, may be formulated as longevity lotion (skin lotion), skin, skin softener, skin toner, astringent, solution, suspension, emulsion, paste, powder, gel, cream, hand cream, hand cleanser, lotion, milk lotion, moisture lotion, nutrition lotion, body lotion, body cleanser, powder, soap, surfactant-containing cleansing, oil, powder foundation, emulsion foundation, wax foundation, spray, and sheet, but is not limited thereto. More specifically, it may be manufactured in the form of softening cosmetic water, nutrition cosmetic water, nutrition cream, massage cream, moisture cream, essence, nutrition essence, eye cream, cleansing cream, cleansing foam, cleansing water, pack, spray, or powder.

The composition in such a formulation may be manufactured according to conventional methods in the art. The compounding amount of additional ingredients such as the moisturizer may be readily selected by a person skilled in the art within a range that does not impair the objects and effects of the present invention.

In the present invention, the composition may be manufactured in the form of a general emulsion formulation and solubilized formulation. Cosmetics in emulsion formulations comprise nutrition cosmetic water, creams, and essences, and cosmetics in solubilized formulations comprise softening cosmetic water. More specifically, the composition of the present invention may be provided in the form of a solution, gel, solid or paste anhydrous product, an emulsion obtained by dispersing an oil phase in a water phase, a suspension, an emulsion, a microemulsion, a microcapsule, a mask pack, microgranules or ionic (liposome) vesicular dispersion type, nonionic vesicular dispersion type, cream, skin, lotion, powder, ointment, spray, paste, pack, cleanser, soap, surfactant-containing cleansing, oil, powder foundation, bath powder, emulsion foundation, wax, foundation, or concealer stick. Additionally, it may be manufactured in the form of a foam or in the form of an aerosol composition further containing a compressed propellant.

When the formulation of the composition is a paste, cream, or gel, carrier components may comprise animal oil, vegetable oil, wax, paraffin, starch, tragacanth, cellulose derivatives, polyethylene glycol, silicone, bentonite, silica, talc, zinc oxide, and the like.

When the formulation of the composition is a powder or spray, carrier components may comprise lactose, talc, silica, aluminum hydroxide, calcium silicate, and polyamide powder, and particularly in the case of a spray, may additionally comprise propellants such as chlorofluorohydrocarbon, propane/butane, and dimethyl ether.

When the formulation of the composition is a solution or emulsion, carrier components may comprise solvents, solubilizers, and emulsifiers, and specifically may comprise water, ethanol, isopropanol, ethyl carbonate, ethyl acetate, benzyl alcohol, benzyl benzoate, propylene glycol, 1,3-butylglycol oil, glycerol aliphatic esters, polyethylene glycol, and fatty acid esters of sorbitan.

When the formulation of the composition is a suspension, carrier components may comprise liquid diluents such as water, ethanol, and propylene glycol; suspending agents such as ethoxylated isostearyl alcohol, polyoxyethylene sorbitol esters, and polyoxyethylene sorbitan esters; and microcrystalline cellulose, aluminum metahydroxide, bentonite, agar, and tragacanth.

When the formulation of the composition is a surfactant-containing cleansing, carrier components may comprise aliphatic alcohol sulfates, aliphatic alcohol ether sulfates, monosesters of sulfosuccinic acid, isethionate, imidazolinium derivatives, methyltaurate, sarcosinate, fatty acid amide ether sulfates, alkylamidobetaine, aliphatic alcohols, fatty acid glycerides, fatty acid diethanolamide, vegetable oils, lanolin derivatives, and ethoxylated glycerol fatty acid esters.

Additionally, compounding components that may be further added are not limited to the above examples, and any of the above components may be compounded within a range that does not impair the objects and effects of the present invention, but preferably are compounded in an amount of 0.01 to 10% by weight, more preferably 0.01 to 5% by weight, based on the total weight.

The cosmetic composition may further comprise, in addition to the components disclosed herein, functional additives and components comprised in general cosmetic compositions, and may further comprise purified water, thickeners, preservatives, stabilizers, solubilizers, surfactants, carriers, fragrances, or combinations thereof, which are conventionally used.

As the carrier, alcohols, oils, surfactants, fatty acids, silicone oils, humectants, moisturizers, viscosity modifiers, emulsifiers, stabilizers, ultraviolet scattering agents, ultraviolet absorbers, colorants, and fragrances may be exemplified. Since compounds or compositions that may be used as the alcohols, oils, surfactants, fatty acids, silicone oils, humectants, moisturizers, viscosity modifiers, emulsifiers, stabilizers, ultraviolet scattering agents, ultraviolet absorbers, colorants, and fragrances are already known in the art, a person skilled in the art may appropriately select and use the corresponding materials or compositions, and additionally, the cosmetic composition may further comprise, as necessary, ultraviolet blockers, antioxidants (butylhydroxyanisole, propyl gallate, erythorbic acid, tocopheryl acetate, butylated hydroxytoluene, etc.), preservatives (methylparaben, butylparaben, propylparaben, phenoxyethanol, imidazolidinyl urea, chlorphenesin, etc.), colorants, pH adjusters (triethanolamine, citric acid, sodium citrate, malic acid, sodium malate, fumaric acid, sodium fumarate, succinic acid, sodium succinate, sodium hydroxide, sodium dihydrogen phosphate, etc.), moisturizers (glycerin, sorbitol, propylene glycol, butylene glycol, hexylene glycol, diglycerin, betaine, glyceryl stearate-26, methylglucose-20, etc.), and lubricants.

In the present invention, the composition may further comprise at least one known component having a skin condition-improving effect and, furthermore, may further comprise at least one material conventionally used in cosmetic compositions. Specifically, the composition may additionally contain adjuvants commonly used in the fields of cosmetology or dermatological science, such as lipids, organic solvents, solubilizers, thickeners and gelling agents, emollients, antioxidants, suspending agents, stabilizers, foaming agents, fragrances, surfactants, water, ionic or non-ionic emulsifiers, fillers, metal-ion sequestering agents and chelating agents, preservatives, vitamins, blockers, humectants, essential oils, dyes, pigments, hydrophilic or lipophilic active agents, lipid vesicles, or any other components conventionally used in cosmetics, and the above components may be introduced in amounts generally used in the field of dermatological science.

In the present invention, the composition may further comprise a composition selected from the group consisting of water-soluble vitamins, oil-soluble vitamins, polymeric peptides, polymeric polysaccharides, and sphingolipids.

In the cosmetic composition of the present invention, other components conventionally blended in cosmetics may be blended as needed in addition to the above essential components, and examples thereof may comprise oily components, moisturizers, emollients, surfactants, organic and inorganic pigments, organic powders, ultraviolet absorbers, preservatives, bactericides, antioxidants, plant extracts, pH adjusters, alcohols, colorants, fragrances, blood-circulation promoters, cooling agents, antiperspirants, and purified water.

### [Modes of the Invention]

Hereinafter, preferred examples are presented to help understand the present invention. However, the following examples are provided only to help understand the present invention more easily, and the contents of the present invention are not limited by the following examples.

### [Examples]

### Example 1. Preparation of long-acting GLP analog, HU020K2FA, for treating metabolic disease

A GLP analog for preventing or treating a metabolic disease was prepared. Specifically, when compared with the GLP-1 sequence, it was designed to share 17 amino acids, and when compared with the GLP-2 sequence, it was designed to share 23 amino acids. Lys, and palmitic acid, which is a C₁₆ fatty acid, were added at the C-terminal, thereby preparing HU020K2FA. Its sequence is shown in Table 1 below.

**[Table 1]**

| SEQ ID NO: | NAME | SEQUENCE |
|---|---|---|
| SEQ ID NO: 1 | HU020K2FA | HGEGTFTSDLSTYLDALAARDFINWLIQTKITDKK(+C16 FA) |
| SEQ ID NO: 2 | HU020 | HGEGTFTSDLSTYLDALAARDFINWLIQTKITD |

### Example 2. Confirmation of excellent effect of activating GLP-1 of HU020K2FA

The GLP-1 activating effects of the long-acting peptide, HU020K2FA, synthesized in Example 1 and HU020 were analyzed. As an analysis method, a cell-based assay was performed to verify the activity of the new GLP-1 peptide. Here, as a cell line, GLP-1R/CHO cells (DiscoverX, 95-0062C2 (product number)) were used. The GLP-1R/CHO cells were reacted with each experimental material to analyze whether this material induces an increase in cAMP. A cAMP concentration was measured using a cAMP assay kit (Discover X, 90-0075- LM10). For GLP-1, EC₅₀ was compared using Exendin-4 (DiscoverX, 92-1115, purity > 95%) as a reference standard. The cells in which a cAMP increase was induced were destroyed and an intracellular cAMP increase was quantified by ELISA to confirm the activity of each experimental material compared to a control. To ensure the accuracy of the activity results, it is desirable to perform at least three repeated experiments on each peptide, so the average value was derived after performing four repeated experiments. Here, the resulting values were compared using GLP-1 Control (Exendin-4), HU020 (non-sustainable), liraglutide, and semaglutide.

As a result, the result of comparing the EC₅₀ of HU020K2FA and HU020 is shown in Table 2 below.

**[Table 2]**

| Administered material | Average (n=4) | |
|---|---|---|
| | (EC₅₀, pM) | Relative to GLP-1 (Exendin-4) % |
| Exendin-4 | 9.6±5.3 | 100.0±55.1 |
| HU020 | 4.8±2.7 | 198.9±112.0 |
| HU020K2FA | 15.1±7.0 | 63.4±29.4 |
| Liraglutide | 32.6±5.4 | 29.4±4.8 |
| Semaglutide | 31.1±7.8 | 30.8±7.7 |

Specifically, cAMP activity was observed in the order of liraglutide (32.6 pM) ≤ semaglutide (31.1 pM) < HU020K2FA (15.1 pM) < Exendin-4 (9.6 pM) < HU020 (4.8 pM). That is, it was confirmed that the GLP-1 activity of HU020K2FA is approximately two-fold higher than positive controls, liraglutide and semaglutide. Based on the facts that Exendin-4, which is a control agonist binding to GLP1R-overexpressing CHO cells, is mainly used for controlling actual blood glucose, and liraglutide and semaglutide exhibit a significantly higher weight loss effect, the results of this experiment suggest that HU020K2FA exhibits at least two-fold *in vitro* receptor-binding activity compared to liraglutide and semaglutide, and HU020K2FA exhibits a significantly higher weight loss effect than Exendin-4, liraglutide, and semaglutide.

### Example 3. Confirmation of excellent obesity treatment effect of HU020K2FA

The obesity treatment effect of the long-acting GLP analog prepared in Example 1 was evaluated. Specifically, an obesity animal model was prepared to confirm the obesity treatment effect, and after administering a certain amount of material, the model was subjected to measurement of a body weight and the change in food intake, and biochemical tests. First, to establish a diet-induced obesity mouse model, C57BL/6 DIO mice were fed a high-fat diet for 12 weeks, resulting in a body weight that was 130% that of the control (DIO model). Afterward, the mice were grouped at a specific time point to determine the average weight of the DIO model (46.2±2.2), followed by drug administration to the mice for 4 weeks. Here, the body weight and food intake were measured daily.

As a result (Table 3 and FIG. 2), it was confirmed that HU020K2FA showed greater weight loss activity than liraglutide, which is equivalent to that of semaglutide. On the other hand, it was confirmed that, despite being administered in the same amount as HU020K2FA, HU020 not only showed an increase in body weight but also an increase in cumulative food intake.

**[Table 3]**

| **Administered material** | **Does (mg/kg/dose)** | **Amount of administered solution (volume, µL)** | **Weight (g)** | **Weight change (%)** | **Cumulative food intake (g)** |
|---|---|---|---|---|---|
| Control | - | 200 | 46.6±3.0 | -0.4±5.3 | 84.5±9.8 |
| Liraglutide | 0.20 | 200 | 40.1±4.9 | -12.5±5.7 | 58.5±2.5 |
| Semaglutide | 0.04 | 200 | 36.6±1.7 | -20.6±2.2 | 50.7±7.4 |
| HU020 | 0.20 | 200 | 47.7±3.0 | +2.2±3.0 | 78.7±18.3 |
| HU020K2FA | 0.20 | 200 | 36.6±4.0 | -19.4±4.7 | 55.3±4.0 |

In addition, after the experiment was completed, blood and serum samples were obtained from each mouse model, and biochemical tests were performed for blood glucose, total cholesterol, high-density lipoprotein (HDL), low-density lipoprotein (LDL), and triglycerides.

As a result (Table 4, and FIG. 3), among all experimental groups, the HU020K2FA-administered group exhibited the highest blood glucose reduction effect, which was confirmed to be significantly higher or similar to those of the actual drugs in use, liraglutide and semaglutide. In addition, the total cholesterol and LDL reduction effects were also confirmed to be superior to the control. Moreover, when HU020 was treated, blood glucose, cholesterol, high-density lipoprotein, low-density lipoprotein, and triglycerides showed significantly higher values, compared to the normal control, indicating that the obesity treatment effect in the animal model was poor.

**[Table 4]**

| **Administered material** | **Blood glucose (mg/dL)** | **Toal cholesterol (mg/dL)** | **High-density lipoprotein (mg/dL)** | **Low-density lipoprotein (mg/dL)** | **Triglyceride (mg/dL)** |
|---|---|---|---|---|---|
| Normal | 211.6±14.1 | 136.9±28.7 | 70.7±11.0 | 12.2±3.2 | 36.2±2.8 |
| Control | 246.8±41.3 | 177.8±22.1 | 80.9±4.6 | 14.3±3.5 | 21.5±5.4 |
| HU020 | 242.8±12.5 | 210.8±26.3 | 95.2±3.8 | 19.8±7.4 | 13.4±2.9 |
| HU020K2FA | 192.0±20.2 | 134.8±10.7 | 76.0±4.0 | 9.8±1.5 | 13.6±4.8 |
| Liraglutide | 209.2±26.7 | 136.2±10.5 | 74.7±2.6 | 9.7±3.3 | 9.6±0.8 |
| Semaglutide | 222.8±22.4 | 122.6±11.0 | 68.5±5.8 | 8.2±3.0 | 12.1±4.5 |

From the above-described results, it was confirmed that the long-acting GLP analog, HU020K2FA, exhibits significantly excellent *in vitro* GLP-1 cAMP activity compared to liraglutide and semaglutide, as well as significantly excellent weight loss effects, cumulative food intake and other biochemical test results also in *in vivo* obesity mouse models.

Particularly, although the cAMP activity of the non-sustainable GLP analog, HU020, of the present invention was the highest among the control, HU020K2FA, liraglutide, and semaglutide, *in vivo* performance was poor; moreover, given the superior obesity treatment activity of HU020K2FA, it was confirmed that *in-vivo* long-acting behavior is very important in enhancing the anti-obesity efficacy of peptide drugs.

Accordingly, the HU020K2FA of the present invention is one of optimized long-acting GLP analogs that can exhibit an obesity treatment effect similar to or significantly superior to that of GLP analog drugs, and can be effectively used as a new long-acting therapeutic agent for obesity.

### Example 4. Additional establishment of long-acting GLP analog for treating obesity

Various materials were further prepared by replacing amino acids, adding lysine, conjugating fatty acids (C16, C18, and C20), or conjugating PEG based on the sequence of the GLP analog prepared in Example 1 (Table 5). Here, palmitic acid was used as C16, octadecanedioic acid was used as C18, and eicosanedioic acid was used as C20. In addition, when a fatty acid was conjugated to lysine, an iso-Glu (gamma-glutamyl) linker used for liraglutide was used. In addition, for HU020M6, a polyethylene glycol(PEG) linker was conjugated to the 20^{th} amino acid to increase peptide solubility and *in vivo* half-life. The conjugate PEG is a 3.4kDa maleimidated PEG, which is a disulfide group (Thiol/SH)-functionalized PEG compound, and a peptide was designed to selectively bind PEG to the disulfide group (SH) at the cysteine residue at position 20.

**[Table 5]**

| SEQ ID NO: | NAME | SEQUENCE |
|---|---|---|
| SEQ ID NO: 3 | HU020M1 | HGEGTFTSDLSTYLDALAARDFINWLIQTKITDKK(+C18 FA) |
| SEQ ID NO: 4 | HU020M2 | HGEGTFTSDLSTYLDALAARDFINWLIQTKIIDKK(+C20 FA) |
| SEQ ID NO: 5 | HU020M3 | HAibEGTFTSDLSTYLDALAARDFINWLIQTKITDKK(+C16 FA) |
| SEQ ID NO: 6 | HU020M4 | HAibEGTFTSDLSTYLDALAARDFINWLIQTKITDKK(+C18 FA) |
| SEQ ID NO: 7 | HU020M5 | HAibEGTFTSDLSTYLDALAARDFINWLIQTKITDKK(+C20 FA) |
| SEQ ID NO: 8 | HU020M6 | HGEGTFTSDLSTYLDALAAC(+mPEG)DFINWLIQTKITDKK(+C16 FA) |
| SEQ ID NO: 9 | HU020M7 | HAibEGTFTSDLSTYLDALAARDFIK(+C18 FA)FAWLIQTKITD |

### Example 5. Confirmation of excellent GLP-1 and GLP-2 activation effect of added GLP analog

The activity of each of the GLP analogs, HU020M1 to HU020M7, represented by SEQ ID NOs: 3 to 9, prepared in Example 4 on GLP-1 and GLP-2 was further analyzed. The analysis method was the same as described in Example 2.

The results are shown in Table 6 below.

**[Table 6]**

| **Name** | **GLP-1 activity** | | **GLP-2 activity** | |
|---|---|---|---|---|
| | **(EC₅₀,pM)** | **Compared to GLP-1 (Exendin-4)** | **(EC₅₀,nM)** | **Compared to GLP-2** |
| GLP-2 (Synthesized) | N.A | N.A | 2.08 | 100 % |
| Exendin-4 (Synthesized) | 7.1 | 100.0% | N.A | N.A |
| Liraglutide (Synthesized) | 25.6 | 27.7% | N.A | N.A |
| Semaglutide (Synthesized) | 29.0 | 24.5% | N.A | N.A |
| HU020 | 4.15 | 171.1% | 650 | 0.32% |
| HU020K2FA | 30.1 | 23.6% | >10000 | ~ 0.02% |
| HU020M1 | 112.6 | 6.3% | >10000 | ~ 0.02% |
| HU020M2 | 461.4 | 1.5% | >10000 | ~ 0.02% |
| HU020M3 | 20.8 | 34.1% | >10000 | ~ 0.02% |
| HU020M4 | 48.1 | 14.8% | >10000 | ~ 0.02% |
| HU020M5 | 236.5 | 3.0% | >10000 | ~ 0.02% |
| HU020M6 | 45.7 | 15.5% | >10000 | ~ 0.02% |
| HU020M7 | 35.2 | 20.2 % | > 50 | < 4.0 % |

Specifically, when comparing the activity on GLP-1 or GLP-2 with that of HU020K2FA of SEQ ID NO: 1, it was found that the GLP-1 activity was maintained in all GLP analogs, except HU020M2 and HU020M5 (C20 fatty acid conjugation). In addition, it was confirmed that SEQ ID NO: 9 (HU020M7) in which the fatty acid conjugation site was changed to position 24 shows the same or higher GLP-1 activity, and further increased GLP-2 activity.

Accordingly, it was identified that *in-vitro* activity varies depending on the type and conjugation site of relevant conjugated fatty acids even when the amino acid sequences are the same.

These results indicate that the peptides of SEQ ID NOs: 1 to 9 showed excellent GLP-1 and GLP-2 activities.

### Example 6. PK animal experiment for added GLP analogs

Mouse PK animal experiments were conducted on SEQ ID NO: 1 prepared in Example 1, and SEQ ID NOs: 3 to 6 prepared in Example 4. Specifically, the concentrations of the drugs in plasma were measured using LC-MS/MS after single subcutaneous administration of semaglutide and candidate materials to mice, and PK-parameters were calculated. As experimental animals, 7-week-old C57BL/6N (male) were used, as experimental groups, HU020K2FA, and HU020M1 to HU020M6 were used, and as a control, semaglutide was used. Each of the experimental groups and the control was subcutaneously administered once to the dorsal area of a total of 12 mice using a 1 mL syringe equipped with a 26-gauge needle at a dose of 0.2 mg/5 mL/kg. After drug administration, approximately 0.04 to 0.05 mL of blood was collected per individual at the time scheduled for the orbital vein (4 mice were alternately sampled), and the blood collection times were 1, 2, 4, 8, 24 ,32 ,48, 56, 72, 96, and 120 hours. All plasma samples were analyzed using a self-developed LC-MS/MS assay. Pharmacokinetic analysis of blood drugs was performed using non-compartmental analysis, and analysis of pharmacokinetic parameters was performed using Phoenix WinNolin 64 (Version. 8.3.5 340).

The results are shown in Table 7 below.

**[Table 7]**

| **Administered material** | **T_{1/2}** | **Tmax** | **Cₘₐₓ** | **AUCₗₐₛₜ** |
|---|---|---|---|---|
| | **h** | **h** | **ng/mL** | **h*ng/mL** |
| Semaglutide | 7.4 | 4.0 | 1126.8 | 15043.4 |
| HU020K2F A | 6.6 | 8.0 | 395.5 | 5958.6 |
| HU020M1 | 13.8 | 12.0 | 746.7 | 24100.1 |
| HU020M2 | 15.9 | 12.0 | 590.5 | 19559.9 |
| HU020M3 | 7.0 | 12.0 | 314.9 | 5071.7 |
| HU020M4 | 10.7 | 8.0 | 810.3 | 23800.9 |
| HU020M5 | 15.2 | 8.0 | 660.7 | 20515.2 |
| HU020M6 | 7.0 | 8.0 | 433.6 | 6508.0 |

Specifically (Table 7 and FIG. 4), it was confirmed that HU020K2FA, which is conjugated with an existing C16 fatty acid, and HU020M3 in which second glycine was substituted with Aib showed a similar half-life to semaglutide, whereas new candidates HU020M1, HU020M2, HU020M4 and HU020M5, which are conjugated with C18 and C20 fatty acids, showed a half-life that is approximately 1.5 to 2.0 times longer than semaglutide.

The above-mentioned results indicate that a metabolic disease treatment effect will be increased with a half-life that is the same as or longer than that of the peptides of SEQ ID NOs: 1 to 9.

### Example 7. Confirmation of excellent anti-obesity effect of added GLP analog

The anti-obesity effects of SEQ ID NO: 1 prepared in Example 1, and SEQ ID NOs: 6 and 7 prepared in Example 4 were analyzed. Here, HU020M4 and HU020M5, which retain GLP-1 activity and have an increased half-life compared to semaglutide, were selected, and comparative experiments were conducted with the control material semaglutide. In addition, as an experimental model, a diet-induced obesity (DIO) mouse model fed a high-fat diet for 3 months was used.

### Example 7-1. Comparison of weight loss and cumulative food intake

To analyze the anti-obesity effect, first, the comparative analysis of weight loss effect and cumulative food intake was conducted. The experimental method was the same as described in Example 3.

The results are shown in Table 8 below and FIG. 5.

**[Table 8]**

| **Administered material** | **Dose (nmol/kg/dose)** | **Administration interval** | **Weight (g)** | **Weight (%) (Normalized to DIO vehicle)** | **Comparison of weight change (%)** | **Food intake (g)** |
|---|---|---|---|---|---|---|
| Vehicle (Normal mice) | - | QD (once daily) | 29.8±1.8 | 64.1±3.8 | 0.0±3.6 | 28.9±5.9 |
| Vehicle (DIO mice) | - | | 46.6±3.5 | 100.0±7.5 | 6.0±4.6 | 25.2±1.9 |
| Semaglutide | 10 | | 37.1±3.9 | 79.7±8.4 | -16.3±4.1 | 15.9±1.6 |
| HU020K2FA | 45 | | 37.5±2.6 | 80.3±4.4 | -16.0±3.2 | 16.0±1.8 |
| HU020M4 | 10 | | 37.4±2.1 | 80.5±8.7 | -15.8±4.0 | 16.4±1.8 |
| HU020M5 | 10 | | 38.0±2.8 | 81.6±6.0 | -14.6±5.1 | 18.3±6.4 |

Specifically, after 4-week administration (once daily (QD)), the weight loss effects of semaglutide, HU020K2FA, HU020M4, and HU020M5 were measured to be almost the same, and it was confirmed that a similar trend to the weight loss effect was observed in cumulative food intake. It is expected that this effect results from the GLP-1 activity of the peptides of the present invention, as well as the effect caused by their increased half-life.

### Example 7-2. Confirmation of fat reduction effect through DEXA imaging

To analyze the anti-obesity effect, body composition analysis was conducted using Dual Energy X-ray Absorptiometry (DEXA). The DEXA imaging system (VET DXA iNSiGHT) is for measuring the bone density and body fat of an experimental animal through dual X-ray transmission, and after the completion of experimental material administration, DEXA fat mass and muscle mass were measured for each individual. Accordingly, body weight was categorized into BMC, lean, and fat, and the fat reduction was analyzed to provide a more objective assessment of the anti-obesity effect according to Example 7-1.

The results are shown in Table 9 below and FIGS. 6 and 7.

**[Table 9]**

| **Material** | **Dose (nmol/kg/dose)** | **Admini stration interval** | **Weight (g)** | **Weight (%) (Normalized to DIO vehicle)** | **Fat (g)** | **Lean (g)** |
|---|---|---|---|---|---|---|
| Vehicle (Normal mice) | - | QD (once daily) | 29.8±1.8 | 64.1±3.8 | 5.8±0.5 | 23.1±1.4 |
| Vehicle (DIO mice) | - | | 46.6±3.5 | 100.0±7.5 | 12.8±1.3 | 32.8±2.6 |
| Semaglutide | 10 | | 37.1±3.9 | 79.7±8.4 | 9.8±1.7 | 26.2±2.7 |
| HU020K2FA | 45 | | 37.5±2.6 | 80.3±4.4 | 9.6±1.0 | 26.5±1.7 |
| HU020M4 | 10 | | 37.4±2.1 | 80.5±8.7 | 9.7±0.7 | 26.6±1.9 |
| HU020M5 | 10 | | 38.0±2.8 | 81.6±6.0 | 9.9±0.9 | 27.3±2.1 |

Specifically, the fat and lean weights in the semaglutide, HU020K2FA, HU020M4, and HU020M5 groups were measured at almost the same level. Particularly, it was confirmed that the HU020M4 and HU020M5 groups show the same effects as the semaglutide group even with the same dose. This result demonstrates that the peptide of the present invention exhibits an excellent anti-obesity effect through amino acid changes, and conjugated fatty acid changes.

### Example 7-3. Confirmation of fat amount reduction effect by tissue

To analyze the anti-obesity effect, the amount of fat by tissue was measured postmortem to further confirm the efficacy of the peptide in obesity. Specifically, the epididymal, mesenteric, inguinal, and perirenal tissues of animals euthanized by decapitation were excised to measure the fat weights for each tissue and then photographed.

The results are shown in Table 10 below and FIG. 8. Specifically, it was confirmed that the amounts of fat by tissue show similar trends to the weight change rate for each drug, such as semaglutide, HU020K2FA, HU020M4, or HU020M5. According to the result, the peptide of the present invention was identified as a material with excellent anti-obesity efficacy because it significantly reduces the fat weights of the epididymal, mesenteric, inguinal, and perirenal regions, which were significantly increased by obesity.

**[Table 10]**

| **Material** | **Dose (nmol/kg/dose)** | **Administration interval** | **Epididymal** | **Mesentric** | **Inguinal** | **Perirenal** |
|---|---|---|---|---|---|---|
| Vehicle (Normal mice) | - | QD (once daily) | 0.86±0.13 | 0.33±0.10 | 0.62±0.12 | 0.12±0.03 |
| Vehicle (DIO mice ) | - | | 1.87±0.37 | 1.69±0.29 | 3.57±0.29 | 0.62±0.12 |
| Semaglutide | 10 | | 2.10±0.26 | 0.74±0.31 | 2.20±0.31 | 0.37±0.12 |
| HU020K2FA | 45 | | 1.71±0.16 | 0.74±0.21 | 2.37±0.21 | 0.33±0.08 |
| HU020M4 | 10 | | 1.75±0.32 | 0.74±0.16 | 2.23±0.16 | 0.31±0.07 |
| HU020M5 | 10 | | 1.76±0.25 | 0.78±0.21 | 2.12±0.21 | 0.32±0.08 |

### Example 7-4. Confirmation of obesity-related factors in plasma

To analyze the anti-obesity effect, mouse plasma was obtained postmortem to measure obesity-related factors in plasma. Here, the analysis method was the same as described in Example 3.

The results are shown in Table 11 below and FIG. 9. Specifically, it was confirmed that the levels of the obesity-related factors in the plasma showed similar trends in all of semaglutide, HU020K2FA, HU020M4, and HU020M5 groups, and particularly, glucose (GLU) in the plasma was reduced in all of the HU020K2FA, HU020M4, and HU020M5 groups, compared to the semaglutide group. Based on these results, the peptide of the present invention reduced all obesity-related factors in plasma, confirming its excellent therapeutic effect against diseases associated with blood fats.

**[Table 11]**

| Administered drug | Dose **nmol/kg/dose** | Administration interval | **GLU (mg/dL)** | **TCHO (mg/dL)** | **TG (mg/dL)** | **LDL (mg/dL)** | **HDL (mg/dL)** | **HbA1c (%)** |
|---|---|---|---|---|---|---|---|---|
| Vehicle (Normal mice ) | - | | 253.0±32.3 | 123.0±13.9 | 57.8±11.8 | 3.6±1.0 | 68.8±6.8 | 4.49±0.15 |
| Vehicle (DIO mice ) | - | QD (once daily) | 308.5±39.3 | 240.1±32.2 | 153.6±51.7 | 11.9±3.9 | 101.0±4.3 | 4.80±0.23 |
| Semaglutide | 10 | | 258.5±16.7 | 166.8±23.0 | 521.4±7.9 | 6.7±1.6 | 86.1±9.3 | 4.59±0.15 |
| HU020K2FA | 45 | | 209.2±9.3 | 198.6±24.3 | 75.1±19.4 | 8.9±9.3 | 100.4±9.3 | 4.39±0.21 |
| HU020M4 | 10 | | 227.8±12.9 | 175.8±28.5 | 49.3±8.8 | 6.4±6.8 | 88.8±6.8 | 4.35±0.22 |
| HU020M5 | 10 | | 214.4±27.1 | 157.3±18.1 | 50.1±9.7 | 6.2±5.8 | 85.7±5.8 | 4.67±0.10 |

### Example 8. Confirmation of excellent anti-obesity effect of added GLP analog in liver

The anti-obesity effect in the liver was analyzed for the same materials used in Example 7. Specifically, liver damage marker (AST/ALT) analysis, liver weight/fatty liver examination, hepatic triglyceride measurement, and liver ORO staining analysis were performed.

### Example 8-1. Liver damage marker (AST/ALT) analysis

To analyze the liver damage markers, AST/ALT, blood biochemical analysis was performed on the blood of a material-treated obese mouse model. Specifically, the collected blood was divided into two tubes, approximately 200 µL of the blood was put into each EDTA tube, and the remaining volume was injected into a vacutainer tube containing a coagulant. Blood collected in an EDTA tube was used to measure glycated hemoglobin on the same day, while the blood collected in the vacutainer tube was left at room temperature for 15 to 20 minutes to coagulate, and then centrifuged for 10 minutes to obtain serum. The serum was used for blood biochemical analysis.

The results are shown in Table 12 below and FIG. 10. Specifically, the AST/ALT levels elevated by obesity were significantly reduced by treatment with HU020K2FA, HU020M4, and HU020M5. Specifically, compared to semaglutide, the peptide of the present invention showed a statistically significant decrease, identifying it as a material with a further improved anti-obesity effect.

**[Table 12]**

| **Administered material** | **Dose (nmol/kg/dose)** | **Administration interval** | **AST (U/L)** | **ALT (U/L)** |
|---|---|---|---|---|
| Vehicle (Normal mice ) | - | QD (once daily) | 56.5±10.3 | 31.2±7.3 |
| Vehicle (DIO mice ) | - | | 136.5±31.8 | 169.7±31.5 |
| Semaglutide | 10 | | 82.2±12.1 | 47.7±11.3 |
| HU020K2FA | 45 | | 68.5±9.2 | 34.7±5.9 |
| HU020M4 | 10 | | 63.3±7.7 | 33.0±8.2 |
| HU020M5 | 10 | | 64.9±5.9 | 33.7±6.0 |

### Example 8-2. Examination of liver weight and steatosis

To examine liver weight and steatosis, the liver weight elevated by obesity induction and the degree of steatosis after liver H&E staining were scored from grades 0 to 3 to compare the drug's hepatic efficacy. First, after the test was completed, the animal's liver was excised on the day of necropsy to confirm the liver efficacy of the drug. After measuring the weight of the excised liver, the intermediate lobes were frozen in liquid nitrogen for the subsequent measurement of triglycerides. The remaining portion of the liver was fixed in 10% neutral buffered formalin and subjected to histopathological examination.

For H&E analysis, the liver tissue fixed in the buffered formalin was put into an automatic tissue processor, embedded in paraffin, and then prepared into a paraffin block. Subsequently, the paraffin block was prepared into a tissue section, which was stained with hematoxylin & eosin (H&E). The stained tissue slide was observed using an optical microscope (BX61, Olympus, Japan) and photographed (DP80, Olympus, Japan).

Histopathological quantification of liver tissue was performed by scoring the degree of steatosis on H&E-stained slides from grades 0 to 3. The contents corresponding to each scoring grade are as follows.

| **Grade** | **Hepatocellular steatosis (H&E)** |
|---|---|
| 0 | < 5% of liver parenchyma* |
| 1 | 5 - 33% of liver parenchyma |
| 2 | 33 - 66% of liver parenchyma |
| 3 | > 66% of liver parenchyma |

| | |
|---|---|
| (* indicates the degree of steatosis in parenchyma cells, and the parenchyma cells in the liver are mainly hepatocytes) | |

The results are shown in Table 13 below and FIG. 11 to 12. Specifically, the liver weights and liver steatosis scores were significantly reduced by treatment with HU020K2FA, HU020M4, and HU020M5, which were confirmed to be superior to semaglutide. Particularly, HU020M4 and HU020M5 exhibited similar effects to semaglutide at the same dose, indicating their exhibit excellent efficacy on the liver.

**[Table 13]**

| **Administered material** | **Dose (nmol/kg/dose)** | **Administrati on interval** | **Liver weight** | **Hepatic steatosis test (Score 0~3)** |
|---|---|---|---|---|
| Vehicle (Normal mice ) | - | QD (once daily) | 1.61±0.36 | 0.18±0.26 |
| Vehicle (DIO mice ) | - | | 2.36±0.33 | 2.77±0.41 |
| Semaglutide | 10 | | 1.51±0.12 | 1.43±0.29 |
| HU020K2FA | 45 | | 1.58±0.10 | 0.97±0.47 |
| HU020M4 | 10 | | 1.39±0.2 | 0.80±0.18 |
| HU020M5 | 10 | | 1.45±0.15 | 0.91±0.25 |

### Example 8-3. Hepatic triglyceride (TG) measurement

To measure hepatic triglyceride levels, triglycerides in liver tissue excised on the necropsy day were analyzed using a measurement kit (Biomax, Cat no. BM-TGR-100).

The results are shown in Table 14 and FIG. 13 below. Specifically, the hepatic triglycerides were significantly reduced when treated with HU020K2FA, HU020M4, and HU020M5. Particularly, it was confirmed that, when HU020M4 and HU020M5 were treated at the same dose as semaglutide, the elevated hepatic triglycerides were reduced to the normal mouse level (vehicle), indicating that the anti-obesity effect was superior to that of semaglutide.

**[Table 14]**

| **Administered material** | **Dose (nmol/kg/dose)** | **Administration interval** | **Liver TG levels (nmol/µ)** |
|---|---|---|---|
| Vehicle (Normal mice) | - | QD (once daily) | 0.60±0.19 |
| Vehicle (DIO mice) | - | | 1.81±0.41 |
| Semaglutide | 10 | | 0.77±0.21 |
| HU020K2FA | 45 | | 0.81±0.11 |
| HU020M4 | 10 | | 0.57±0.18 |
| HU020M5 | 10 | | 0.61±0.18 |

### Example 8-4. ORO analysis

Finally, to confirm the anti-obesity effect in the liver, liver Oil Red O staining analysis was performed. ORO staining is a staining method used to detect triglycerides (fat) in adipose-related diseases, and used to differentiate fat embolism, liposarcoma, hepatic steatosis, and lipid accumulation disorders. In addition, it can also detect lipid complexes, such as phospholipids. For ORO analysis, liver tissue was processed in 30% sucrose in a refrigerator for 3 days to prepare frozen tissue sections, which were used to prepare OCT-embedded frozen blocks. Cryosections were prepared and stained with ORO.

The results are shown in Table 15 below and FIGS. 14 and 15. Specifically, the hepatic ORO positive area (%) was significantly reduced after treatment with HU020K2FA, HU020M4, and HU020M5, and almost no triglycerides, which appear orange to deep red, were observed to the naked eye. Particularly, it was confirmed that this effect is superior to that of semaglutide, regardless of the type and dose of HU020K2FA, HU020M4, and HU020M5.

**[Table 15]**

| **Administered material** | **Dose (nmol/kg/dose)** | **Administration interval** | **ORO positive area (%)** |
|---|---|---|---|
| Vehicle (Normal mice) | - | QD (once daily) | 0.26±0.19 |
| Vehicle (DIO mice) | - | | 5.95±1.57 |
| Semaglutide | 10 | | 2.08±0.75 |
| HU020K2FA | 45 | | 1.41±0.66 |
| HU020M4 | 10 | | 0.90±0.21 |
| HU020M5 | 10 | | 1.17±0.66 |

### Example 9. Confirmation of sustained anti-obesity effect of added GLP analog

The administration interval was extended using HU020M4 exhibiting the best efficacy with conventional QD (once daily) administration, and it was analyzed whether a sustained anti-obesity effect was observed. Here, the same experimental methods as previously used were applied, and the possibility of Q2D (once every two days) administration was tested in the same DIO obese mice with the control semaglutide.

### Example 9-1. Comparison of body weight reduction effect and cumulative food intake

The results are shown in Table 16 below and FIG. 16.

**[Table 16]**

| **Administered material** | **Dose nmol/kg/dose** | **Administration interval** | **Weight (g)** | **Weight (%)** (Normalized to DIO vehicle) | **Comparison of weight change (%)** | **Food intake (g)** |
|---|---|---|---|---|---|---|
| Vehicle (Normal mice) | - | | 28.0±2.0 | 62.4±4.5 | +1.4±2.3 | 44.6±5.1 |
| Vehicle (DIO mice) | - | Q2D (once every two days) | 44.8±2.7 | 100.0±6.1 | -6.2±3.4 | 32.0±2.3 |
| Semaglutide | 10 | | 39.0±2.7 | 87.0±6.1 | -18.5±4.1 | 23.0±3.4 |
| Semaglutide | 25 | | 36.9±2.0 | 72.3±4.4 | -22.9±2.7 | 20.5±2.5 |
| HU020M4 | 10 | | 36.9±4.8 | 72.2±8.5 | -23.2±7.2 | 21.2±1.3 |
| HU020M4 | 25 | | 34.7±3.8 | 77.3±3.1 | -27.7±3.0 | 18.4±1.5 |

Specifically, in all administered groups compared to G2 (obese mice), body weights were statistically significantly reduced (P < 0.05). When semaglutide and HU020M4 were administered at the same dose of 25 nmol/kg once every two days (Q2D), particularly, HU020M4 showed significant body weight loss efficacy (P < 0.05). In addition, the semaglutide 25 nmol/kg/Q2D administered group and the HU020M4 10 nmol/kg/Q2D administered group showed the same level of the body weight loss efficacy as that of the HU020M4 25 nmol/kg/Q2D administered group (P > 0.05).

The above-mentioned results demonstrated that, due to the half-life of HU020M4, which is 1.5-fold longer than that of semaglutide, HU020M4 showed the same level of body weight reduction efficacy even at half the drug concentration, and a higher body weight reduction efficacy was observed when HU020M4 was administered at the same dose of 25 nmol/kg/Q2D.

Consequently, even when administered once daily, as well as once every two days, the HU020M4 of the present invention exhibited excellent anti-obesity activity compared to semaglutide, proved by animal experiment data.

### Example 9-2. Confirmation of levels of obesity-related factors in plasma

The analysis results are shown in Table 17 below and FIG 17. Specifically, the levels of obesity-related factors in plasma were reduced by treatment with both semaglutide and HU020M4, whereas the HU020M4-induced reduction effect was equal to or greater than that for most factors.

**[Table 17]**

| Administered drug | | Dose **nmol/kg/dose** | Administration interval | **GLU (mg/dL)** | **TCHO (mg/dL)** | **TG (mg/dL)** | **HDL (mg/dL)** | **LDL (mg/dL)** |
|---|---|---|---|---|---|---|---|---|
| Buffer | Vehicle (Normal mice ) | - | | 227.8±15.9 | 75.3±8.0 | 82.1±18.4 | 45.0±7.6 | 15.9±11.2 |
| | Vehicle (DIO mice ) | - | Q2D (once every two days) | 295.9±39.2 | 191.0±25.4 | 123.3±84.5 | 115.0±15.5 | 55.9±17.5 |
| Control | Semaglutide | 10 | | 235.5±21.6 | 135.0±24.8 | 51.6±10.4 | 82.5±16.0 | 42.2±10.8 |
| | Semaglutide | 25 | | 230.0±15.1 | 142.1±23.0 | 50.7±15.3 | 80.0±12.2 | 49.5±15.4 |
| Huonslab candidate substance | HU020M4 | 10 | | 219.3±29.3 | 132.1±19.8 | 56.2±7.0 | 68.6±11.8 | 53.9±9.8 |
| | HU020M4 | 25 | | 214.8±24.3 | 122.4±18.4 | 45.5±13.4 | 67.5±17.9 | 45.8±11.4 |

### Example 9-3. Analysis of liver damage markers AST/ALT

The analysis results are shown in Table 18 below and FIG. 18. Specifically, the levels of obesity-related factors in plasma were reduced by treatment with both semaglutide and HU020M4. Particularly, the HU020M4 treatment exhibited better reduction effects on all components such as GLU, TCHO, TG, LDL, and HDL, compared to semaglutide.

**[Table 18]**

| **Administered material** | **Dose (nmol/kg/dose)** | **Administratio n interval** | **AST (U/L)** | **ALT (U/L)** |
|---|---|---|---|---|
| Vehicle (Normal mice) | - | Q2D (Once every two days) | 39.1±2.9 | 32.9±4.1 |
| Vehicle (DIO mice) | - | | 79.0±24.0 | 84.2±51.9 |
| Semaglutide | 10 | | 65.0±10.5 | 55.0±17.3 |
| Semaglutide | 25 | | 59.5±14.5 | 53.3±23.6 |
| HU020M4 | 10 | | 58.7±11.4 | 38.3±16.6 |
| HU020M4 | 25 | | 52.9±6.9 | 33.8±8.6 |

The foregoing description of the present invention is intended for illustrative purposes, and it will be understood by those skilled in the art that various modifications can be made thereto in other specific forms without departing from the technical spirit or essential features of the present invention. Therefore, the embodiments described above should be understood as illustrative in all respects and not restrictive.

### [Industrial Applicability]

The present invention relates to a pharmaceutical composition for preventing or treating obesity, the composition comprising a GLP (glucagon like peptide) analog as an active ingredient, and the GLP analog of the present invention, as an analog of GLP-1 and GLP-2, was confirmed to exhibit excellent effects in preventing or treating a metabolic disease due to an increased half-life resulting in an extended duration of in vivo activity, and specifically, in view of the facts that it reduced the body weight and fat of a subject, decreased the levels of blood glucose, total blood cholesterol, high-density lipoprotein, low-density lipoprotein, and triglycerides as blood lipid factors, and further decreased hepatic ASL and AST levels, liver weight, a hepatic steatosis grade, and a hepatic triglyceride level, the peptide of the present invention may be usefully utilized as an excellent agent for preventing or treating a metabolic disease, and thus has industrial applicability.

## Claims

1. A peptide represented by Formula I below:
[Formula I] HX₂EGX₅FTX₈DX₁₀X₁₁X₁₂X₁₃X₁₄X₁₅X₁₆X₁₇X₁₈X₁₉X₂₀X₂₁FIX₂₄X₂₅LX₂₇ X₂₈X₂₉X₃₀X₃₁X₃₂X₃₃X₃₄X₃₅X₃₆X₃₇X₃₈X₃₉X₄₀X₄₁X₄₂X₄₃
In Formula I,
X₂ is Gly or Aib;
X₅ is Thr or Ser;
X₈ is Ser or Asp;
X₁₀ is Leu or Val;
X₁₁ is Ser or Asn;
X₁₂ is Thr or Ser;
X₁₃ is Tyr or Ile;
X₁₄ is Leu or Met;
X₁₅ is Asp or Glu;
X₁₆ is any one selected from the group consisting of Ala, Gly, and Asn;
X₁₇ is Leu or Gln;
X₁₈ and X₁₉ are Ala, or His;
X₂₀ is Arg, or Cys;
X₂₁ is Asp or Glu;
X₂₄ is Asn or Lys;
X₂₅ is Trp or Thr;
X₂₇ is Ile or Val;
X₂₈ is Gln or Lys;
X₂₉ is Thr or Gly;
X₃₀ is Lys or Arg;
X₃₁ is Ile or absent;
X₃₂ is Thr or absent;
X₃₃ is Asp or absent; and
X₃₄ to X₄₃ are absent or Lys.

2. The peptide of claim 1, wherein X₅ is Thr; X₈, and X₁₁ are Ser; X₁₂ is Thr; X₁₃ is Tyr; X₁₄ is Leu; X₁₅ is Asp; X₁₆ is Ala; X₁₇ is Leu; X₁₉ is Ala; X₂₁ is Asp; X₂₅ is Trp; X₂₇ is Ile; X₂₈ is Gln; X₂₉ is Thr; X₃₀ is Lys; X₃₁ is Ile; X₃₂ is Thr; X₃₃ is Asp, X₃₄ to X₄₃, or X₃₆ to X₄₃ are absent.

3. The peptide of claim 1 or 2, wherein X₁₀ is Leu, X₁₈ is Ala, and X₃₄ and X₃₅ are Lys.

4. The peptide of any one of claims 1 to 3, wherein when X₂₀ is Cys, Cys is conjugated with polyethylene glycol (PEG).

5. The peptide of any one of claims 1 to 4, wherein a fatty acid binds to any one or more amino acids selected from the group consisting of X₂₄ and X₃₅ to X43.

6. The peptide of claim 5, wherein the fatty acid is a C₁₆ to C₂₀ fatty acid.

7. The peptide of claim 6, wherein the fatty acid is any one selected from the group consisting of palmitic acid, octadecanedioic acid, eicosanedioic acid, margaric acid, heptadecanedioic acid, hexadecanedioic acid, stearic acid, nonadecylic acid, nonadecanedioic acid, and arachidic acid.

8. The peptide of any one of claims 1 to 7, wherein the peptide comprises any one selected from the group consisting of amino acid sequences represented by SEQ ID NOs: 1 to 9.

9. A pharmaceutical composition for preventing or treating a metabolic disease, comprising the peptide of any one of claims 1 to 8 as an active ingredient.

10. The pharmaceutical composition of claim 9, wherein the metabolic disease is any one or more selected from the group consisting of obesity, diabetes, non-alcoholic fatty liver disease, hepatic steatosis, dyslipidemia, atherosclerosis, insulin resistance syndrome, and their complications.

11. The pharmaceutical composition of claim 9 or 10, wherein the peptide has a long-acting form.

12. The pharmaceutical composition of any one of claims 9 to 11, wherein the composition is administered by any one route selected from the group consisting of subcutaneous, intravenous, and oral administrations.

13. The pharmaceutical composition of any one of claims 9 to 12, wherein the composition activates GLP-1 and GLP-2.

14. The pharmaceutical composition of any one of claims 9 to 13, wherein the composition is **characterized by** any one or more selected from the group consisting of the following:
a) reducing the body weight and fat of an individual;
b) lowering blood glucose, total blood cholesterol, high-density lipoprotein, low-density lipoprotein, and triglycerides; and
c) decreasing hepatic ASL and AST levels, liver weight, a hepatic steatosis grade, and a hepatic triglyceride level.

15. A kit for preventing or treating a metabolic disease, comprising a composition comprising the peptide of any one of claims 1 to 8 as an active ingredient; and instructions.

16. A food composition for preventing or improving a metabolic disease, comprising the peptide of any one of claims 1 to 8 as an active ingredient.

17. The food composition of claim 16, wherein the food is a health functional food.

18. A cosmetic composition for preventing or improving obesity, comprising the peptide of any one of claims 1 to 8 as an active ingredient.

19. A method of preventing, alleviating, or treating a metabolic disease, comprising:
administering a pharmaceutically effective amount of a composition comprising the peptide of any one of claims 1 to 8 as an active ingredient to an individual in need thereof.

20. A use of a composition comprising the peptide of any one of claims 1 to 8 as an active ingredient for preventing, alleviating, or treating a metabolic disease.

21. A use of a composition comprising the peptide of any one of claims 1 to 8 as an active ingredient for preparing a preparation for preventing, alleviating, or treating a metabolic disease.
